# EUROPEAN PATENT APPLICATION

(11) **EP 2 135 956 A1**
(43) Date of publication of application: **23.12.2009**
(21) Application number: 08722748.4
(22) Date of filing: 25.03.2008
(51) Int. Cl.: C12P 13/02, C12N 15/52, C12R 1/85

(54) **SPHINGOLIPID HAVING ENDOPLASMIC RETICULUM LOCALIZATION SIGNAL ATTACHED THERETO, AND METHOD FOR PRODUCTION OF CERAMIDE IN TRANSFORMED CELL USING 4-DESATURASE**

(30) Priority: 30.03.2007 JP 2007094762; 12.06.2007 JP 2007155037
(71) Applicant: Suntory Holdings Limited, Kita-ku, Osaka-shi Osaka 530-8203 (JP); Hiroshima University, Higashi-Hiroshima-shi Hiroshima 739-8511 (JP)
(72) Inventor: KODAMA, Yukiko, Mishima-gun Osaka 618-8503 (JP); OKUHARA, Hiroaki, Mishima-gun Osaka 618-8503 (JP); FUNATO, Koichi, Higashi-Hiroshima-shi Hiroshima 739-8528 (JP)
(74) Representative: Vossius & Partner
(86) International application number: PCT/JP2008/055485
(87) International publication number: WO 2008/120604

(57) **Abstract**

The present invention provides a method for producing human ceramide in cells such as yeast cells.

The method of the present invention comprises:
1) introducing, by transformation of the yeast cell, an endoplasmic reticulum localization signal-attached sphingolipid Δ4-desaturase gene (DES1m) in which a nucleotide sequence encoding a yeast endoplasmic reticulum localization signal is added to the 3'-end of the sphingolipid Δ4-desaturase gene (DES1); and
2) abolishing, by transformation of the yeast cell, the expression of the yeast sphinganine C4-hydroxylase gene (SUR2).

SEQUENCE LISTING
<110> Suntory Limited

<120> Method for producing ceramide in transformed cells using the
sphingolipid-Δ4-desaturase attached with an endoplasmic reticulum
localization signal

<130> R2735 EP S3

<140> EP 08 72 2748.4
<141> 2008-03-25

<150> JP 2007-094762
<151> 2007-03-30
<150> JP 2007-155037
<151> 2007-06-12
<160> 43

<210> 1
<211> 972
<212> DNA
<213> Homo sapiens
<400> 1
atggggagcc gcgtctcgcg ggaagacttc gagtgggtct acaccgacca gccgcacgcc 60
gaccggcgcc gggagatcct ggcaaagtat ccagagataa agtccttgat gaaacctgat 120
cccaatttga tatggattat aattatgatg gttctcaccc agttgggtgc attttacata 180
gtaaaagact tggactggaa atgggtcata tttggggcct atgcgtttgg cagttgcatt 240
aaccactcaa tgactctggc tattcatgag attgcccaca atgctgcctt tggcaactgc 300
aaagcaatgt ggaatcgctg gtttggaatg tttgctaatc ttcctattgg gattccatat 360
tcaatttcct ttaagaggta tcacatggat catcatcggt accttggagc tgatggcgtc 420
gatgtagata ttcctaccga ttttgagggc tggttcttct gtaccgcttt cagaaagttt 480
atatgggtta ttcttcagcc tctcttttat gcctttcgac ctctgttcat caaccccaaa 540
ccaattacgt atctggaagt tatcaatacc gtggcacagg tcacttttga cattttaatt 600
tattactttt tgggaattaa atccttagtc tacatgttgg cagcatcttt acttggcctg 660
ggtttgcacc caatttctgg acattttata gctgagcatt acatgttctt aaagggtcat 720
gaaacttact catattatgg gcctctgaat ttacttacct tcaatgtggg ttatcataat 780
gaacatcatg atttccccaa cattcctgga aaaagtcttc cactggtgag gaaaatagca 840
gctgaatact atgacaacct ccctcactac aattcctgga taaaagtact gtatgatttt 900
gtgatggatg atacaataag tccctactca agaatgaaga ggcaccaaaa aggagagatg 960
gtgctggagt aa 972

<210> 2
<211> 323
<212> PRT
<213> Homo sapiens
<400> 2
Met Gly Ser Arg Val Ser Arg Glu Asp Phe Glu Trp Val Tyr Thr
1               5                   10                  15
Asp Gln Pro His Ala Asp Arg Arg Arg Glu Ile Leu Ala Lys Tyr
                20                  25                  30
Pro Glu Ile Lys Ser Leu Met Lys Pro Asp Pro Asn Leu Ile Trp
                35                  40                  45
Ile Ile Ile Met Met Val Leu Thr Gln Leu Gly Ala Phe Tyr Ile
                50                  55                  60
Val Lys Asp Leu Asp Trp Lys Trp Val Ile Phe Gly Ala Tyr Ala
                65                  70                  75
Phe Gly Ser Cys Ile Asn His Ser Met Thr Leu Ala Ile His Glu
                80                  85                  90
Ile Ala His Asn Ala Ala Phe Gly Asn Cys Lys Ala Met Trp Asn
                95                  100                 105

Arg Trp Phe Gly Met Phe Ala Asn Leu Pro Ile Gly Ile Pro Tyr
                110                 115                 120
Ser Ile Ser Phe Lys Arg Tyr His Met Asp His His Arg Tyr Leu
                125                 130                 135
Gly Ala Asp Gly Val Asp Val Asp Ile Pro Thr Asp Phe Glu Gly
                140                 145                 150
Trp Phe Phe Cys Thr Ala Phe Arg Lys Phe Ile Trp Val Ile Leu
                155                 160                 165
Gln Pro Leu Phe Tyr Ala Phe Arg Pro Leu Phe Ile Asn Pro Lys
                170                 175                 180
Pro Ile Thr Tyr Leu Glu Val Ile Asn Thr Val Ala Gln Val Thr
                185                 190                 195
Phe Asp Ile Leu Ile Tyr Tyr Phe Leu Gly Ile Lys Ser Leu Val
                200                 205                 210
Tyr Met Leu Ala Ala Ser Leu Leu Gly Leu Gly Leu His Pro Ile
                215                 220                 225
Ser Gly His Phe Ile Ala Glu His Tyr Met Phe Leu Lys Gly His
                230                 235                 240
Glu Thr Tyr Ser Tyr Tyr Gly Pro Leu Asn Leu Leu Thr Phe Asn
                245                 250                 255
Val Gly Tyr His Asn Glu His His Asp Phe Pro Asn Ile Pro Gly
                260                 265                 270
Lys Ser Leu Pro Leu Val Arg Lys Ile Ala Ala Glu Tyr Tyr Asp
                275                 280                 285
sn Leu Pro His Tyr Asn Ser Trp Ile Lys Val Leu Tyr Asp Phe
                290                 295                 300
Val Met Asp Asp Thr Ile Ser Pro Tyr Ser Arg Met Lys Arg His
                305                 310                 315
Gln Lys Gly Glu Met Val Leu Glu
                320         323

<210>  3
<211>  987
<212>  DNA
<213>  Homo sapiens
<400>  3
   atggggagcc gcgtctcgcg ggaagacttc gagtcggtct acaccgacca gccgcacgcc    60
   gaccggcgcc gggagatcct ggcaaagtat ccagagataa agtccttgat gaaacctgat   120
   cccaatttga tatggattat aattatgatg gttctcaccc agttgggtgc attttacata   180
   gtaaaagact tggactggaa atgggtcata tttggggcct atgcgtttgg cagttgcatt   240
   aaccactcaa tgactctggc tattcatgag attgcccaca atgctgcctt tggcaactgc   300
   aaagcaatgt ggaatcgctg gtttggaatg tttgctaatc ttcctattgg gattccatat   360
   tcaatttcct ttaagaggta tcacatggat catcatcggt accttggagc tgatggcgtc   420
   gatgtagata ttcctaccga ttttgagggc tggttcttct gtaccgcttt cagaaagttt   480
   atatgggtta ttcttcagcc tctcttttat gcctttcgac ctctgttcat caaccccaaa   540
   ccaattacgt atctggaagt tatcaatacc gtggcacagg tcacttttga cattttaatt   600
   tattactttt tgggaattaa atccttagtc tacatgttgg cagcatcttt acttggcctg   660
   ggtttgcacc caatttctgg acattttata gctgagcatt acatgttctt aaagggtcat   720
   gaaacttact catattatgg gcctctgaat ttacttacct tcaatgtggg ttatcataat   780
   gaacatcatg atttccccaa cattcctgga aaaagtcttc cactggtgag gaaaatagca   840
   gctgaatact atgacaacct ccctcactac aattcctgga taaaactact gtatgatttt   900
   gtgatggatg atacaataag tccctactca agaatgaaga ggcaccaaaa aggagagatg   960
   gtgctggagg taaagaaaga gaaataa    987

<210>  4
<211>  328
<212>  PRT
<213>  Homo sapiens
<400>  4
Met Gly Ser Arg Val Ser Arg Glu Asp Phe Glu Trp Val Tyr Thr

1               5                   10                  15
Asp Gln Pro His Ala Asp Arg Arg Arg Glu Ile Leu Ala Lys Tyr
                20                  25                  30
Pro Glu Ile Lys Ser Leu Met Lys Pro Asp Pro Asn Leu Ile Trp
                35                  40                  45
Ile Ile Ile Met Met Val Leu Thr Gln Leu Gly Ala Phe Tyr Ile
                50                  55                  60
Val Lys Asp Leu Asp Trp Lys Trp Val Ile Phe Gly Ala Tyr Ala
                65                  70                  75
Phe Gly Ser Cys Ile Asn His Ser Met Thr Leu Ala Ile His Glu
                80                  85                  90
Ile Ala His Asn Ala Ala Phe Gly Asn Cys Lys Ala Met Trp Asn
                95                  100                 105
Arg Trp Phe Gly Met Phe Ala Asn Leu Pro Ile Gly Ile Pro Tyr
                110                 115                 120
Ser Ile Ser Phe Lys Arg Tyr His Met Asp His His Arg Tyr Leu
                125                 130                 135
Gly Ala Asp Gly Val Asp Val Asp Ile Pro Thr Asp Phe Glu Gly
                140                 145                 150
Trp Phe Phe Cys Thr Ala Phe Arg Lys Phe Ile Trp Val Ile Leu
                155                 160                 165
Gln Pro Leu Phe Tyr Ala Phe Arg Pro Leu Phe Ile Asn Pro Lys
                170                 175                 180
Pro Ile Thr Tyr Leu Glu Val Ile Asn Thr Val Ala Gln Val Thr
                185                 190                 195
Phe Asp Ile Leu Ile Tyr Tyr Phe Leu Gly Ile Lys Ser Leu Val
                200                 205                 210
Tyr Met Leu Ala Ala Ser Leu Leu Gly Leu Gly Leu His Pro Ile
                215                 220                 225
Ser Gly His Phe Ile Ala Glu His Tyr Met Phe Leu Lys Gly His
                230                 235                 240
Glu Thr Tyr Ser Tyr Tyr Gly Pro Leu Asn Leu Leu Thr Phe Asn
                245                 250                 255
Val Gly Tyr His Asn Glu His His Asp Phe Pro Asn Ile Pro Gly
                260                 265                 270
Lys Ser Leu Pro Leu Val Arg Lys Ile Ala Ala Glu Tyr Tyr Asp
                275                 280                 285
sn Leu Pro His Tyr Asn Ser Trp Ile Lys Val Leu Tyr Asp Phe
                290                 295                 300
Val Met Asp Asp Thr Ile Ser Pro Tyr Ser Arg Met Lys Arg His
                305                 310                 315
Gln Lys Gly Glu Met Val Leu Glu Val Lys Lys Glu Lys
                320                 325         328

<210>  5
<211>  1050
<212>  DNA
<213>  Saccharomyces cerevisiae
<400>  5
atgaacgtaa catcgaatgc aactgcagcc ggttcctttc cactagcatt tggtctcaag    60
acctcatttg ggtttatgca ctatgccaag gcccctgcca ttaatttacg ccccaaggaa   120
tccttgctgc cggaaatgag tgatggtgtg ctggccttgg ttgcgccggt tgttgcctac   180
tgggcgttgt ctggtatatt ccatgtaata gacactttcc atctggctga gaagtacaga   240
attcatccga gcgaagaggt tgccaagagg aacaaggcgt cgagaatgca tgttttcctt   300
gaactgattc tacaacatat catacagacc attgttggcc ttatctttat gcacttcgag   360
ccgatctaca tgactgggtt tgaagaaaat gccatgtgga agcttcgtgc agaccttcct   420
cggattattc cagatgccgc tatttattac ggctatatgt acggaatgtc cgctttgaag   480
atctttgcag gcttttattt cgttgataca tggcaatact tttgcataga ttgatgcat   540
atgaataaga ccttatacaa atggttccac tctgttcatc atgaactata cgtgccatat   600
gcttacgctg ctcttttcaa caatcctgtt gagggcttct tgttagatac tttgggaacc   660

ggtattgcca tgacgttaac tcatttgact cacagagagc aaatcattct ttttaccttt 720
gccaccatga agactgtcga tgaccactgt gggtatgctt tgccacttga cccattccaa 780
tggcttttcc ctaataacgc tgtctatcac gatatccacc accagcaatt tggtatcaag 840
acgaactttg ctcaaccatt tttcactttc tgggacaatt tgttccaaac taactttaaa 900
gggtttgaag aatatcaaaa gaagcaaaga cgtgtcacca tcgacaagta caaagagttt 960
ttgcaagaga gagaattgga aaagaaggag aaactcaaaa acttcaaagc tatgaatgct 1020
gctgaaaatg aagtaaagaa agagaaataa 1050

<210> 6
<211> 349
<212> PRT
<213> Saccharomyces cerevisiae
<400> 6
Met Asn Val Thr Ser Asn Ala Thr Ala Ala Gly Ser Phe Pro Leu
1               5                   10                  15
Ala Phe Gly Leu Lys Thr Ser Phe Gly Phe Met His Tyr Ala Lys
                20                  25                  30
Ala Pro Ala Ile Asn Leu Arg Pro Lys Glu Ser Leu Leu Pro Glu
                35                  40                  45
Met Ser Asp Gly Val Leu Ala Leu Val Ala Pro Val Val Ala Tyr
                50                  55                  60
Trp Ala Leu Ser Gly Ile Phe His Val Ile Asp Thr Phe His Leu
                65                  70                  75
Ala Glu Lys Tyr Arg Ile His Pro Ser Glu Glu Val Ala Lys Arg
                80                  85                  90
Asn Lys Ala Ser Arg Met His Val Phe Leu Glu Val Ile Leu Gln
                95                  100                 105
His Ile Ile Gln Thr Ile Val Gly Leu Ile Phe Met His Phe Glu
                110                 115                 120
Pro Ile Tyr Met Thr Gly Phe Glu Glu Asn Ala Met Trp Lys Leu
                125                 130                 135
Arg Ala Asp Leu Pro Arg Ile Ile Pro Asp Ala Ala Ile Tyr Tyr
                140                 145                 150
Gly Tyr Met Tyr Gly Met Ser Ala Leu Lys Ile Phe Ala Gly Phe
                155                 160                 165
Leu Phe Val Asp Thr Trp Gln Tyr Phe Leu His Arg Leu Met His
                170                 175                 180
Met Asn Lys Thr Leu Tyr Lys Trp Phe His Ser Val His His Glu
                185                 190                 195
Leu Tyr Val Pro Tyr Ala Tyr Gly Ala Leu Phe Asn Asn Pro Val
                200                 205                 210
Glu Gly Phe Leu Leu Asp Thr Leu Gly Thr Gly Ile Ala Met Thr
                215                 220                 225
Leu Thr His Leu Thr His Arg Glu Gln Ile Ile Leu Phe Thr Phe
                230                 235                 240
Ala Thr Met Lys Thr Val Asp Asp His Cys Gly Tyr Ala Leu Pro
                245                 250                 255
Leu Asp Pro Phe Gln Trp Leu Phe Pro Asn Asn Ala Val Tyr His
                260                 265                 270
Asp Ile His His Gln Gln Phe Gly Ile Lys Thr Asn Phe Ala Gln
                275                 280                 285
Pro Phe Phe Thr Phe Trp Asp Asn Leu Phe Gln Thr Asn Phe Lys
                290                 295                 300
Gly Phe Glu Glu Tyr Gln Lys Lys Gln Arg Arg Val Thr Ile Asp
                305                 310                 315
Lys Tyr Lys Glu Phe Leu Gln Glu Arg Glu Leu Glu Lys Lys Glu
                320                 325                 330
Lys Leu Lys Asn Phe Lys Ala Met Asn Ala Ala Glu Asn Glu Val
                335                 340                 345
Lys Lys Glu Lys

349

<210>  7
<211>  1155
<212>  DNA
<213>  Saccharomyces cerevisiae
<400>  7
atgtcgacta atacttccaa gactttggaa ctgttttcaa aaaagacggt acaagaacac    60
aatactgcca atgactgctg ggtcacttat caaaacagaa agatttatga cgtgaccagg   120
tttttgagcg aacaccctgg tggtgacgag tccatcttgg actatgctgg taaggacatt   180
actgagatca tgaaagactc agatgtgcat gaacacagcg actccgcgta tgaaatcctt   240
gaggacgaat atttgattgg ttacttggca actgacgaag aggcagcgag attgttgact   300
aacaagaacc ataaggttga agtgcagttg tcagctgacg gtactgagtt tgactccact   360
acttttgtaa aggagttgcc cgccgaggag aaactaagta ttgctacgga ctacagtaac   420
gactacaaaa agcataaatt tttggatctg aaccgtcctt tgctgatgca gattctgcgt   480
agtgatttca agaaagattt ttacgttgac caaatccata gaccaagaca ttacggtaag   540
gggtctgccc cgctatttgg taatttcttg gaaccattaa ctaaaacagc ttggtgggtt   600
gttccagttg cttggttgcc tgtagttgtg taccacatgg gtgttgcttt gaagaacatg   660
aaccagctat ttgcatgttt cttgttctgt gtcggtgtct ttgtttggac tttgattgaa   720
tacggtcttc accgtttcct atttcatttc gatgattggt tacctgaaag taacatcgca   780
ttcgccacac attttctact acatggttgc catcattact tgcccatgga caagtaccgt   840
ttagttatgc cacctactct gttcgtcatc ctttgtgctc cattttacaa gttggtattt   900
gctctgctgc cactttattg ggcttacgct ggttttgctg gcggtctttt cggttatgtc   960
tgttatgacg aatgtcattt cttcttgcac cactctaaat tgcctccctt catgcgtaag  1020
ttgaaaaaat atcacctgga acatcattat aaaaactacc aactgggatt tggcgtcaca  1080
tcctgctttt gggacgaagt ttttggcacc tacttaggcc ccgatgcccc attgtccaaa  1140
atgaaatatg aataa  1155

<210>  8
<211>  384
<212>  PRT
<213>  Saccharomyces cerevisiae
<400>  8
Met Ser Thr Asn Thr Ser Lys Thr Leu Glu Leu Phe Ser Lys Lys
1               5                   10                  15
Thr Val Gln Glu His Asn Thr Ala Asn Asp Cys Trp Val Thr Tyr
                20                  25                  30
Gln Asn Arg Lys Ile Tyr Asp Val Thr Arg Phe Leu Ser Glu His
                35                  40                  45
Pro Gly Gly Asp Glu Ser Ile Leu Asp Tyr Ala Gly Lys Asp Ile
                50                  55                  60
Thr Glu Ile Met Lys Asp Ser Asp Val His Glu His Ser Asp Ser
                65                  70                  75
Ala Tyr Glu Ile Leu Glu Asp Glu Tyr Leu Ile Gly Tyr Leu Ala
                80                  85                  90
Thr Asp Glu Glu Ala Ala Arg Leu Leu Thr Asn Lys Asn His Lys
                95                  100                 105
Val Glu Val Gln Leu Ser Ala Asp Gly Thr Glu Phe Asp Ser Thr
                110                 115                 120
Thr Phe Val Lys Glu Leu Pro Ala Glu Glu Lys Leu Ser Ile Ala
                125                 130                 135
Thr Asp Tyr Ser Asn Asp Tyr Lys Lys His Lys Phe Leu Asp Leu
                140                 145                 150
Asn Arg Pro Leu Leu Met Gln Ile Leu Arg Ser Asp Phe Lys Lys
                155                 160                 165
Asp Phe Tyr Val Asp Gln Ile His Arg Pro Arg His Tyr Gly Lys
                170                 175                 180
Gly Ser Ala Pro Leu Phe Gly Asn Phe Leu Glu Pro Leu Thr Lys
                185                 190                 195
Thr Ala Trp Trp Val Val Pro Val Ala Trp Leu Pro Val Val Val

                200                 205                 210
Tyr His Met Gly Val Ala Leu Lys Asn Met Asn Gln Leu Phe Ala
                215                 220                 225
Cys Phe Leu Phe Cys Val Gly Val Phe Val Trp Thr Leu Ile Glu
                230                 235                 240
Tyr Gly Leu His Arg Phe Leu Phe His Phe Asp Asp Trp Leu Pro
                245                 250                 255
Glu Ser Asn Ile Ala Phe Ala Thr His Phe Leu Leu His Gly Cys
                260                 265                 270
His His Tyr Leu Pro Met Asp Lys Tyr Arg Leu Val Met Pro Pro
                275                 280                 285
Thr Leu Phe Val Ile Leu Cys Ala Pro Phe Tyr Lys Leu Val Phe
                290                 295                 300
Ala Leu Leu Pro Leu Tyr Trp Ala Tyr Ala Gly Phe Ala Gly Gly
                305                 310                 315
Leu Phe Gly Tyr Val Cys Tyr Asp Glu Cys His Phe Phe Leu His
                320                 325                 330
His Ser Lys Leu Pro Pro Phe Met Arg Lys Leu Lys Lys Tyr His
                335                 340                 345
Leu Glu His His Tyr Lys Asn Tyr Gln Leu Gly Phe Gly Val Thr
                350                 355                 360
Ser Trp Phe Trp Asp Glu Val Phe Gly Thr Tyr Leu Gly Pro Asp
                365                 370                 375
Ala Pro Leu Ser Lys Met Lys Tyr Glu
                380                 384

<210>  9
<211>  954
<212>  DNA
<213>  Saccharomyces cerevisiae
<400>  9
atgctgttca gctggcctta tccagaagcc ccgattgaag gttattgggg caagccaact     60
tctctgattg attggtgcga ggagaattat gtcgtatccc cctatattgc agaatggtca    120
aatactatta ccaatagtat attcttaatg accgccttct attctacata tagcgcttgg    180
cgtaataagt tagaaacaag gtatatattg atacgaatgg ggttctcgct ggttggtatt    240
ggttcgtggt tatttcatat gactttacag tatcgttatc aattgctaga cgaactacca    300
atgctgtatg cgaccatcat cccatcgtgg agtatttttg cagaaactca agaaatcttg    360
attaaggatg agaagaaaag gaaggaaagc tcatttagaa tccaaatggt catttctttt    420
atcatgtgtg gtatagtcac cattttaacc tggatttacg ttgtcgtcca aaagccagca    480
attttccaag tcctttatgg tatattgacg cttctagttg tggttctttc tggctggctg    540
acctactatc acgttcatga ttcatttgca aagaaaaatc tttttattac tatggttatg    600
ggcatgattc cttttgtcat tgggttcatt tgctggcaac tagatattca cctgtgttct    660
ttttggatct atatccggag aacatatttg gccctgccat taggtgttct attggaactg    720
catgcttggt ggcatctttt gaccggtact ggtgtctata tcttcgtcgt gtatttgcaa    780
tatttgagaa tattaaccca tggaaatcca aatgacttct tatttatatg gaggtgggga    840
tttttccctg agctggtaag aaagggctta ccgattggta cttcttattc actggagtat    900
ctggggccaa ttgtaaatac acaggtagat gatcaaacaa aaaagaataa ctaa    954

<210>  10
<211>  317
<212>  PRT
<213>  Saccharomyces cerevisiae
<400>  10
Met Leu Phe Ser Trp Pro Tyr Pro Glu Ala Pro Ile Glu Gly Tyr
1               5                   10                  15
Trp Gly Lys Pro Thr Ser Leu Ile Asp Trp Cys Glu Glu Asn Tyr
                20                  25                  30
Val Val Ser Pro Tyr Ile Ala Glu Trp Ser Asn Thr Ile Thr Asn
                35                  40                  45
Ser Ile Phe Leu Met Thr Ala Phe Tyr Ser Thr Tyr Ser Ala Trp

50                      55                      60
Arg Asn Lys Leu Glu Thr Arg Tyr Ile Leu Ile Gly Met Gly Phe
                65                      70                      75
Ser Leu Val Gly Ile Gly Ser Trp Leu Phe His Met Thr Leu Gln
                80                      85                      90
Tyr Arg Tyr Gln Leu Leu Asp Glu Leu Pro Met Leu Tyr Ala Thr
                95                      100                     105
Ile Ile Pro Ser Trp Ser Ile Phe Ala Glu Thr Gln Glu Ile Leu
                110                     115                     120
Ile Lys Asp Glu Lys Lys Arg Lys Glu Ser Ser Phe Arg Ile Gln
                125                     130                     135
Met Val Ile Ser Phe Ile Met Cys Gly Ile Val Thr Ile Leu Thr
                140                     145                     150
Trp Ile Tyr Val Val Val Gln Lys Pro Ala Ile Phe Gln Val Leu
                155                     160                     165
Tyr Gly Ile Leu Thr Leu Leu Val Val Val Leu Ser Gly Trp Leu
                170                     175                     180
Thr Tyr Tyr His Val His Asp Ser Phe Ala Lys Lys Asn Leu Phe
                185                     190                     195
Ile Thr Met Val Met Gly Met Ile Pro Phe Val Ile Gly Phe Ile
                200                     205                     210
Cys Trp Gln Leu Asp Ile His Leu Cys Ser Phe Trp Ile Tyr Ile
                215                     220                     225
Arg Arg Thr Tyr Leu Ala Leu Pro Leu Gly Val Leu Leu Glu Leu
                230                     235                     240
His Ala Trp Trp His Leu Leu Thr Gly Thr Gly Val Tyr Ile Phe
                245                     250                     255
Val Val Tyr Leu Gln Tyr Leu Arg Ile Leu Thr His Gly Asn Pro
                260                     265                     270
Asn Asp Phe Leu Phe Ile Trp Arg Trp Gly Phe Phe Pro Glu Leu
                275                     280                     285
Val Arg Lys Gly Leu Pro Ile Gly Thr Ser Tyr Ser Leu Glu Tyr
                290                     295                     300
Leu Gly Pro Ile Val Asn Thr Gln Val Asp Asp Glu Thr Lys Lys
                305                     310                     315
Asn Asn
    317

<210>  11
<211>  44
<212>  DNA
<213>  Artificial sequence
<220>
<221>  source
<223>  /note= "Description of artificial sequence: Designed
       oligonucleotide as a primer for PCR amplification"
<400>  11
ccttctctag aggatccatg gggagccgcg tctcgcggga agac    44

<210>  12
<211>  45
<212>  DNA
<213>  Artificial sequence
<220>
<221>  source
<223>  /note= "Description of artificial sequence: Designed
       oligonucleotide as a primer for PCR amplification"
<400>  12
ccttcgaatt ccccgggcca ggggagcttc tgagcatcac tcgtc    45

<210> 13
<211> 66
<212> DNA
<213> Artificial sequence
<220>
<221> source
<223> /note= "Description of artificial sequence: Designed
      oligonucleotide as a primer for PCR amplification"
<400> 13
ctccggcttc tgcggttttt cttagtcttt ccgcaccaat tttcacagga attcccgggg
atccgg   66

<210> 14
<211> 68
<212> DNA
<213> Artificial sequence
<220>
<221> source
<223> /note= "Description of artificial sequence: Designed
      oligonucleotide as a primer for PCR amplification"
<400> 14
ggataataaa tacaaacgtg ggaagtcgga gacattgcct ttacccagca agctagcttg
gctgcagg    68

<210> 15
<211> 31
<212> DNA
<213> Artificial sequence
<220>
<221> source
<223> /note= "Description of artificial sequence: Designed
      oligonucleotide as a primer for PCR amplification"
<400> 15
ctccggcttc tgcggttttt cttagtcttt c   31

<210> 16
<211> 28
<212> DNA
<213> Artificial sequence
<220>
<221> source
<223> /note= "Description of artificial sequence: Designed
      oligonucleotide as a primer for PCR amplification"
<400> 16
ggaagtcgga gacattgcct ttacccag   28

<210> 17
<211> 27
<212> DNA
<213> Artificial sequence
<220>
<221> source
<223> /note= "Description of artificial sequence: Designed
      oligonucleotide as a primer for PCR amplification"
<400> 17
cgaattcagc cgaaaacagt cttgctt   27

<210> 18
<211> 30
<212> DNA

<213>  Artificial sequence
<220>
<221>  source
<223>  /note= "Description of artificial sequence: Designed
       oligonucleotide as a primer for PCR amplification"
<400>  18
ctccatgtcg cttaccaccg cttttagtgc   30

<210>  19
<211>  31
<212>  DNA
<213>  Artificial sequence
<220>
<221>  source
<223>  /note= "Description of artificial sequence: Designed
       oligonucleotide as a primer for PCR amplification"
<400>  19
cgctatactg cagcctcgtc caaaattgtca   31

<210>  20
<211>  27
<212>  DNA
<213>  Artificial sequence
<220>
<221>  source
<223>  /note= "Description of artificial sequence: Designed
       oligonucleotide as a primer for PCR amplification"
<400>  20
cgaattcttg ccaacctgat ctgtgaa   27

<210>  21
<211>  25
<212>  DNA
<213>  Artificial sequence
<220>
<221>  source
<223>  /note= "Description of artificial sequence: Designed
       oligonucleotide as a primer for PCR amplification"
<400>  21
gcgctgcata catagacata tacac   25

<210>  22
<211>  25
<212>  DNA
<213>  Artificial sequence
<220>
<221>  source
<223>  /note= "Description of artificial sequence: Designed
       oligonucleotide as a primer for PCR amplification"
<400>  22
atacgcgatc gctgttaaaa ggaca   25

<210>  23
<211>  26
<212>  DNA
<213>  Artificial sequence
<220>
<221>  source
<223>  /note= "Description of artificial sequence: Designed
       oligonucleotide as a primer for PCR amplification"

<400>  23
cgaattcccc agaggcaaag atgtta   26

<210>  24
<211>  39
<212>  DNA
<213>  Artificial sequence
<220>
<221>  source
<223>  /note= "Description of artificial sequence: Designed
       oligonucleotide as a primer for PCR amplification"
<400>  24
tggatggcac ggatccgaaa ggcacacctg tcattatgg   39

<210>  25
<211>  34
<212>  DNA
<213>  Artificial sequence
<220>
<221>  source
<223>  /note= "Description of artificial sequence: Designed
       oligonucleotide as a primer for PCR amplification"
<400>  25
tgtgcctttc ggatccgtgc catccatttg aatc   34

<210>  26
<211>  30
<212>  DNA
<213>  Artificial sequence
<220>
<221>  source
<223>  /note= "Description of artificial sequence: Designed
       oligonucleotide as a primer for PCR amplification"
<400>  26
cgaattcctt ttatgatggg agtaactgct   30

<210>  27
<211>  42
<212>  DNA
<213>  Artificial sequence
<220>
<221>  source
<223>  /note= "Description of artificial sequence: Designed
       oligonucleotide as a primer for PCR amplification"
<400>  27
aaacctgcag gcccagaggc aaagatgtta agttagattt tc   42

<210>  28
<211>  42
<212>  DNA
<213>  Artificial sequence
<220>
<221>  source
<223>  /note= "Description of artificial sequence: Designed
       oligonucleotide as a primer for PCR amplification"
<400>  28
tttcctgcag gcttttatga tgggagtaac tgctgcatgt ct   42

<210>  29
<211>  28

<212>  DNA
<213>  Artificial sequence
<220>
<221>  source
<223>  /note= "Description of artificial sequence: Designed
       oligonucleotide as a primer for PCR amplification"
<400>  29
taagatctga catggaggcc cagaatac   28

<210>  30
<211>  28
<212>  DNA
<213>  Artificial sequence
<220>
<221>  source
<223>  /note= "Description of artificial sequence: Designed
       oligonucleotide as a primer for PCR amplification"
<400>  30
taagatctcg cacttaactt cgcatctg   28

<210>  31
<211>  20
<212>  DNA
<213>  Artificial sequence
<220>
<221>  source
<223>  /note= "Description of artificial sequence: Designed
       oligonucleotide as a primer for PCR amplification"
<400>  31
ctcgtcctct gaaccaaagc   20

<210>  32
<211>  25
<212>  DNA
<213>  Artificial sequence
<220>
<221>  source
<223>  /note= "Description of artificial sequence: Designed
       oligonucleotide as a primer for PCR amplification"
<400>  32
ggtaaataga acttttatgt gccgc   25

<210>  33
<211>  28
<212>  DNA
<213>  Artificial sequence
<220>
<221>  source
<223>  /note= "Description of artificial sequence: Designed
       oligonucleotide as a primer for PCR amplification"
<400>  33
aacctgcagg tggaattctg agcggata   28

<210>  34
<211>  29
<212>  DNA
<213>  Artificial sequence
<220>
<221>  source

<223>  /note= "Description of artificial sequence: Designed
       oligonucleotide as a primer for PCR amplification"
<400>  34
ttcctgcagg ttttcccagt cacgacgtt   29

<210>  35
<211>  48
<212>  DNA
<213>  Artificial sequence
<220>
<221>  source
<223>  /note= "Description of artificial sequence: Designed
       oligonucleotide as a primer for PCR amplification"
<400>  35
atggtgctgg aggtaaagaa agagaaataa atatcattag tgccaaag   48

<210>  36
<211>  48
<212>  DNA
<213>  Artificial sequence
<220>
<221>  source
<223>  /note= "Description of artificial sequence: Designed
       oligonucleotide as a primer for PCR amplification"
<400>  36
taatgatatt tatttctctt tctttacctc cagcaccatc tctccttt   48

<210>  37
<211>  2147
<212>  DNA
<213>  Artificial sequence
<220>
<221>  source
<223>  /note= "Description of artificial sequence:  Designed DNA to obtain
       the human sphingo-Δ4-desaturase attached with the endoplasmic
       reticula localization signal"
<400>  37
aacctgcagg tggaattgtg agcggataac aatttcacac aggaaacagc tatgaccatg   60
attacgccaa gcgcgcaatt aaccctcact aaagggaaca aaagctggag ctcatattgt  120
tttcttcacc aaccatcagt tcataggtcc attctcttag cgcaactaca gagaacaggg  180
gcacaaacag gcaaaaaacg ggcacaacct caatggagtg atgcaacctg cctggagtaa  240
atgatgacac aaggcaattg acccacgcat gtatctatct cattttctta caccttctat  300
taccttctgc tctctctgat ttggaaaaag ctgaaaaaaa aggttgaaac cagttccctg  360
aaattattcc cctacttgac taataagtat ataaagacgg taggtattga ttgtaattct  420
gtaaatctat ttcttaaact tcttaaattc tacttttata gttagtcttt tttttagttt  480
taaaacacca gaacttagtt tcgacggatt ctagaactag tggatccatg gggagccgcg  540
tctcgcggga agacttcgag tgggtctaca ccgaccagcc gcacgccgac cggcgccggg  600
agatcctggc aaagtatcca gagataaagt ccttgatgaa acctgatccc aatttgatat  660
ggattataat tatgatggtt ctcacccagt tgggtgcatt ttacatagta aaagacttgg  720
actggaaatg ggtcatattt gcggcctatg cgtttggcag ttgcattaac cactcaatga  780
ctctggctat tcatgagatt gcccacaatg ctgcctttgg caactgcaaa gcaatgtgga  840
atcgctggtt tggaatgttt gctaatcttc ctattgggat tccatattca atttccttta  900
agaggtatca catggatcat catcggtacc ttggagctga tggcgtcgat gtagatattc  960
ctaccgattt tgagggctgg ttcttctgta ccgctttcag aaagtttata tgggttattc 1020
ttcagcctct cttttatgcc tttcgacctc tgttcatcaa ccccaaacca attacgtatc 1080
tggaagttat caataccgtg gcacaggtca cttttgacat tttaatttat tactttttgg 1140
gaattaaatc cttagtctac atgttggcag catctttact tggcctgggt ttgcacccaa 1200
tttctggaca ttttatagct gagcattaca tgttcttaaa gggtcatgaa acttactcat 1260
attatgggcc tctgaattta cttaccttca atgtgggtta tcataatgaa catcatgatt 1320
tccccaacat tcctggaaaa agtcttccac tggtgaggaa aatagcagct gaatactatg 1380

acaacctccc tcactacaat tcctggataa aagtactgta tgattttgtg atggatgata 1440
caataagtcc ctactcaaga atgaagaggc accaaaaagg agagatggtg ctggaggtaa 1500
agaaagagaa ataaatatca ttagtgccaa agggattctt ctccaaaact ttagatgata 1560
aaatggaatt tttgcattat taaacttgag accagtgatg ctcagaagct cccctggccc 1620
gggcagctgt gattgattga gtcgacttgg ttgaacacgt tgccaaggct taagtgaatt 1680
tactttaaat cttgcattta aataaatttt ctttttatag ctttatgact tagtttcaat 1740
ttatatacta ttttaatgac attttcgatt caytgattga aagcttatcg ataccgtcga 1800
cctcgagtca tgtaattagt tatgtcacgc ttacattcac gccctccccc cacatccgct 1860
ctaaccgaaa aggaaggagt tagacaacct gaagtctagg tccctattta tttttttata 1920
gttatgttag tattaagaac gttatttata tttcaaattt ttcttttttt tctgtacaga 1980
cgcgtgtacg catgtaacat tatactgaaa accttgcttg agaaggtttt gggacgctcg 2040
aaggctttaa tttgcggccg gtacccaatt cgccctatag tgagtcgtat tacgcgcgct 2100
cactggccgt cgttttacaa cgtcgtgact gggaaaacct gcaggaa 2147

<210> 38
<211> 20
<212> DNA
<213> Artificial sequence
<220>
<221> source
<223> /note= "Description of artificial sequence: Designed
      oligonucleotide as a primer for PCR amplification"
<400> 38
tccagcacca tctctccttt 20

<210> 39
<211> 20
<212> DNA
<213> Artificial sequence
<220>
<221> source
<223> /note= "Description of artificial sequence: Designed
      oligonucleotide as a primer for PCR amplification"
<400> 39
agtgggtcta caccgaccag

<210> 40
<211> 1732
<212> DNA
<213> Homo sapiens
<400> 40
gccgccgccg ccgccgccac ctctgagcag ccggctggga gcgagagccg acagctagtc 60
tgcaagccac cgctgtcgcc atggggagcc ccgtctcgcg ggaagacttc gagtgggtct 120
acaccgacca gccgcacgcc gaccggcgcc gggagatcct ggcaaagtat ccagagataa 180
agtccttgat gaaacctgat cccaatttga tatggattat aattatgatg gttctcaccc 240
agttgggtgc attttacata gtaaaagact tggactggaa atgggtcata tttggggcct 300
atgcgtttgg cagttgcatt aaccactcaa tgactctggc tattcatgag attgcccaca 360
atgctgcctt tggcaactgc aaagcaatgt ggaatcgctg gtttggaatg tttgctaatc 420
ttcctattgg gattccatat tcaatttcct ttaagaggta tcacatggat catcatcggt 480
accttggagc tgatggcgtc gatgtagata ttcctaccga ttttgagggc tggttcttct 540
gtaccgcttt cagaaagttt atatgggtta ttcttcagcc tctcttttat gcctttcgac 600
ctctgttcat caaccccaaa ccaattacgt atctggaagt tatcaatacc gtggcacagg 660
tcacttttga cattttaatt tattactttt tgggaattaa atccttagtc tacatgttgg 720
cagcatcttt acttggcctg ggtttgcacc caatttctgg acattttata gctgagcatt 780
acatgttctt aaacggtcat gaaacttact catattatgg gcctctgaat ttacttacct 840
tcaatgtggg ttatcataat gaacatcatg atttccccaa cattcctgga aaaagtcttc 900
cactggtgag gaaaatagca gctgaatact atgacaacct ccctcactac aattcctgga 960
taaaagtact gtatgatttt gtgatgcatg atacaataag tccctactca agaatgaaga 1020
ggcaccaaaa aggagagatg gtgctgcagt aaatatcatt agtgccaaag ggattcttct 1080
ccaaaacttt agatgataaa atggaatttt tgcattatta aacttgagac cagtgatgct 1140

cagaagctcc cctggcacaa tttcagagta agagctcggt gataccaaga agtgaatctg 1200
gcttttaaac agtcagcctg actctgtact gctcagtttc actcacagga aacttgtgac 1260
ttgtgtatta tcgtcattga ggatgtttca ctcatgtctg tcattttata agcatatcat 1320
ttaaaaagct tctaaaaagc tatttcgcca ggcacggtgg ctcatgccta taatcccagc 1380
actttgggag gccaaggtgg gtggatcacc tgaggtcagg agttcgagac cagcctggcc 1440
aacacggtga aaccccatct ctactaaaaa tgcaaaaatt agccgggcgt ggcggcacat 1500
gcctgtaatc ccagctacat gggaggctga ggtgggagaa ttgcttgaac ccaggagccg 1560
gaggcagagg ctgcagtcac ccaagattgt gccactgcac tccaccctgg gcaacagagc 1620
aagaccccat ctcaaaaata aataaatata tataaaaaat aaaaagctat ttctagttta 1680
tttcactata aagttttgct ttattaaaaa gctaataaac agctattaat cc 1732

<210> 41
<211> 1420
<212> DNA
<213> Saccharomyces cerevisiae
<400> 41
ctccggcttc tgcggttttt cttagtcttt ccgcaccaat tttcacagaa aaatttcttt 60
ttgggttact aaaaatttct tgtttgctat tgtcttgatt tatttcactc tttcttcatc 120
ctgctgcgct gttgttgccg catttttcgt ttcgtgtttt gggattataa aagaagtaac 180
gatttacata tcttctattc ccttgcattt actccgggtc ttcgtcttta ctgtttggtc 240
tgggtatttc taacattcta gtccgaagag ggtgtatacg aaaagaaaat atacgatgaa 300
cgtaacatcg aatgcaactg cagccggttc ctttccacta gcatttggtc tcaagacctc 360
atttgggttt atccactatg ccaaggcccc tgccattaat ttacgcccca aggaatcctt 420
gctgccggaa atgagtgatg gtgtgctggc cttggttgcg ccggttgttg cctactgggc 480
gttgtctggt atattccatg taatagacac tttccatctg gctgagaagt acagaattca 540
tccgagcgaa gaggttgcca agaggaacaa ggcgtcgaga atgcatgttt tccttgaagt 600
gattctacaa catatcatac agaccattgt tggccttatc tttatgcact tcgagccgat 660
ctacatgact gggtttgaag aaaatgccat gtggaagctt cgtgcagacc ttcctcgcat 720
tattccagat gccgctattt attacggcta tatgtacgga atgtccgctt tgaagatctt 780
tgcaggcttt ttattcgttg atacatggca atactttttg catagattga tgcatatgaa 840
taagacctta tacaaatggt tccactctgt tcatcatgaa ctatacgtgc catatgctta 900
cggtgctctt ttcaacaatc ctgttgaggg cttcttgtta gatactttgg gaaccggtat 960
tgccatgacg ttaactcatt tgactcacag agagcaaatc attcttttta cctttgccac 1020
catgaagact gtcgatgacc actgtgggta tgctttgcca cttgacccat tccaatggct 1080
tttccctaat aacgctgtct atcacgatat ccaccaccag caatttggta tcaagacgaa 1140
ctttgctcaa ccatttttca ctttctggga caatttgttc caaactaact ttaaagggtt 1200
tgaagaatat caaaagaagc aaagacgtgt caccatcgac aagtacaaag agtttttgca 1260
agagagagaa ttggaaaaga aggagaaact caaaaacttc aaagctatga atgctgctga 1320
aaatgaagta aagaaagaga aataaccctt ttgcatacct cccgttcaat tgctgggtaa 1380
aggcaatgtc tccgacttcc cacgtttgta tttattatcc 1420

<210> 42
<211> 1920
<212> DNA
<213> Saccharomyces cerevisiae
<400> 42
   cctgaaaagg cttcagcgct gcatacatag acatatacac atcacacgta catacacgaa 60
   aagatagctt tcatgctcac aaagctacgt tccccacaga atatttccca ggacaacagg 120
   gaaagttgga caaacaccaa acttctcata atacaatagc cgaaaacagt cttgcttttg 180
   ataaagagag ctttctgcgg ccggaagaaa atgtgcctat tccgcggggc cgttccgcgc 240
   agaaactacc gcacgacgtc ggaaatataa cggggttttg cgcggattta ctgacgtctg 300
   acatcttttc gcagttaatt tttttttcaa tttggaaccg tataagaaca gaggtattag 360
   atagtagaag ctattgtgtc tattgtcttt gattagtttt tttttctatt gttatcgtgg 420
   caggcactaa aagcggtggt aagctaaaac tagtacgaag atgtcgacta atacttccaa 480
   gactttggaa ctgttttcaa aaaagacggt acaagaacac aatactgcca atgactgctg 540
   ggtcacttat caaaacagaa agatttatga cgtgaccagg tttttgagcg aacaccctgg 600
   tggtgacgag tccatcttgg actatgctgg taaggacatt actgagatca tgaaagactc 660
   agatgtgcat gaacacagcg actccgcgta tgaaatcctt gaggacgaat atttgattgg 720
   ttacttggca actgacgaag aggcagcgag attgttgact aacaagaacc ataaggttga 780
   agtgcagttg tcagctgacg gtactgagtt tgactccact acttttgtaa aggagttgcc 840
   cgccgaggag aaactaagta ttgctacgga ctacagtaac gactacaaaa agcataaatt 900
   tttggatctg aaccgtcctt tgctgatgca gattctgcgt agtgatttca agaaagattt 960
   ttacgttgac caaatccata gaccaagaca ttacggtaag ggtctgcccc cgctatttgg 1020
   taatttcttg gaaccattaa ctaaaacagc ttggtcggtt gttccagttg cttggttgcc 1080
   tgtagttgtg taccacatgg gtgttgcttt gaagaacatg aaccagctat ttgcatgttt 1140
   cttgttctgt gtcggtgtct ttgtttggac tttgattgaa tacggtcttc accgtttcct 1200
   atttcatttc gatgattggt tacctgaaag taacatcgca ttcgccacac attttctact 1260
   acatggttgc catcattact tgcccatgga caagtaccgt ttagttatgc cacctactct 1320
   gttcgtcatc ctttgtgctc cattttacaa gttggtattt gctctgctgc cactttattg 1380
   ggcttacgct ggttttgctg gcggtctttt cggttatgtc tgttatgacg aatgtcattt 1440
   cttcttgcac cactctaaat tgcctccctt catgcgtaag ttgaaaaaat atcacctgga 1500
   acatcattat aaaaactacc aactgggatt tggcgtcaca tcctggtttt gggacgaagt 1560
   ttttggcacc tacttaggcc ccgatgcccc attgtccaaa atgaaatatg aataattatt 1620
   ggtcagcctc gtccaaaatt gtcaacctag gaaaaacaac attacgagca atgaaatctt 1680
   gagcaaagtt cttttctgcg acatatttat ttcaaatctt tatttatatt attatatgta 1740
   actacgtata tatacataca catggaatgg aatcatagat gaaatcgatg aacaagcacg 1800
   ttatttctta aacgaatatc caatcgatga cgactatacg aaaaagcggc ctcaaaaaag 1860
   aaccgaattg acaggtacat ttttctttca cagatcaggt tggcaaaaaa atactaaccg 1920

<210> 43
<211> 2160
<212> DNA
<213> Saccharomyces cerevisiae
<400> 43
gacacaatat tggtttactt ccatggtctc ctaacgcacg aggcatgttg actcgtcctc 60
tgaaccaaag cacggacagg attaagagtg atccaacttt caagtcgtta catttggata 120
atctcgaaga agaacaaaag gaaattataa atcgtgtgga aaaggtgtcg aaggacaaaa 180
aagtctcgat ggctatgctc tccattgcat gggttttgca taaaggatgt caccctattg 240
tggcattgaa cactacagca agagtacacg aagcgattgc cgcactacaa gtaactctaa 300
cagaagaaga gataaagtac ctcgaggagc cctacaaacc ccagaggcaa agatgttaag 360
ttagattttc atgggcccac tgatatatag aaggctaaat aacgagaatc atatatactg 420
taacatgaca cctgctgatt tttactgatc gtagccattc ggctcatatt acttcatgtc 480
ccttttttaa atccctcgtt cccggagagt ggcatagcat gtccgatagc gtacgccaac 540
ctacagaatc aggggatcag atttcctgaa agcaacttat ccatcagttt ctttttataa 600
tgccataatg acaggtgtgc ctttcttatt ttacttcgct tgctttcttt ctattgcatg 660
cttcaatctt tgaatatttg ctaatactga aaaaaaaaaa ggaaataact atctggttta 720
tctgcattgt tcttcgtttt tcaattcaag aatttaagca aaaggatata tcatagaacc 780
tagtgaattt ttaagaaagt aagataaaga aaaaaatcaa atgctgttca gctggcctta 840
tccagaagcc ccgattgaag gttattcggg caagccaact tctctgattg attggtgcga 900
ggagaattat gtcgtatccc cctatattgc agaatggtca aatactatta ccaatagtat 960
attcttaatg accgccttct attctacata tagcgcttgg cgtaataact tagaaacaag 1020
gtatatattg ataggaatgg ggttctcgct cggttggtatt ggttcgtggt tatttcatat 1080
gactttacag tatcgttatc aattgctaga cgaactacca atgctgtatg cgaccatcat 1140
cccatcgtgg agtatttttg cagaaactca agaaatcttg attaaggatg agaagaaaag 1200
gaaggaaagc tcatttagaa tccaaatggt catttctttt atcatgtgtg gtatagtcac 1260
cattttaacc tggatttacg ttgtcgtcca aaagccagca attttccaag tcctttatgg 1320
tatattgacg cttctagttg tggttctttc tggctggctg acctactatc acgttcatga 1380
ttcatttgca aagaaaaatc tttttattac tatggttatg ggcatgattc cttttgtcat 1440
tgggttcatt tgctggcaac tagatattca cctgtgttct ttttggatct atatccgcag 1500
aacatatttg gccctgccat taggtgttct attggaactg catgcttggt ggcatctttt 1560
gaccggtact ggtgtctata tcttcgtcgt gtatttgcaa tatttgagaa tattaaccca 1620
tggaaatcca aatgacttct tatttatatg gaggtgggga tttttccctg agctggtaag 1680
aaacggctta ccgattggta cttcttattc actggagtat ctggggccaa ttgtaaatac 1740
acacgtagat gatgaaacaa aaaagaataa ctaaggagtg atttgtgcc atccattga 1800
atcttcaaaa tatatgtacg tatatgttat gtatgtatat aaattgttgt acgtgcaatt 1860
gaccgaacgt gtatatttgt atatagatat gtgacaaact aaacagctta cacttcgttc 1920
cttcccttct gttcaccttt ttttattatg cctgactatt ctaaaattta tgtttatgtg 1980
gcgtaaaata tctttcttct gaaaacacat gcagcagtta ctcccatcat aaaaggaatt 2040
aataaatgaa tagtataaac aattatttac ttaaatatat ttaaatttct gcttggaaaa 2100
ccggccatgt cggcggcaca taaaagttct atttaccttt aaattctttt cgacatgtat 2160

## Description

### TECHNICAL FIELD

The present application claims priority based on Japanese Patent Application No. 2007-94762 filed on March 30, 2007 and Japanese Patent Application No. 2007-155037 filed on June 12, 2007.

The present invention relates to methods for producing human ceramide in cells such as yeast cells.

### BACKGROUND ART

A tissue known as stratum corneum exists on the outermost layer of the skin, which has a moisturizing function for retaining moisture as well as a barrier function for protecting the skin against external stimulation. The stratum corneum consists of keratinocytes, natural moisturizing factors and intercellular lipids, among which ceramides account for approximately one-half of the total intercellular lipids and play a crucial role for these functions. For example, a common characteristic of atopic dermatitis and senile xerosis is a significant deterioration of moisturizing ability, which is known to mainly result from decreased ceramide levels due to lipid metabolic enzyme abnormalities. Ceramides have also been shown to enhance barrier function, provide whitening effect and inhibit melanogenesis. Ceramides can be externally supplied.

J Invest Dermatol. 96:523-526, 1991 (Non-patent document 1) and Arch Dermatol Res. 283:219-223, 1991 (Non-patent document 2) disclose "decreased ceramide levels in atopic dermatitis and senile xerosis"; J Dermatol Sci. 1:79-83, 1990 (Non-patent document 3) and Acta Derm Venereol. 74:337-340, 1994 (Non-patent document 4) disclose "decreased ceramide levels and lipid metabolic enzyme abnormalities"; Contact Dermatitis. 45:280-285, 2001 (Non-patent document 5) and J Eur Acad Dermatol Venereol. 16:587-594, 2002 (Non-patent document 6) disclose "restoration of barrier function by ceramides"; and Cell Signal 14:779-785, 2002 (Non-patent document 7) discloses "inhibition of melanogenesis by ceramides."

Recently, ceramides have attracted great attention for use in medicines for skin diseases associated with dry sensitive skin or in cosmetics or health and/or cosmeceutical foods. In fact, a number of products such as cosmetics and food or supplements containing ceramides have already been commercialized, and the market for ceramide materials is continuing to grow.

Ceramide materials of animal origin such as cow were conventionally used, but are currently replaced by those of plant origin such as rice, wheat, soybean and potato because of problems of infections. A recent basic study (J. Clin. Invest. 112:1372-1382, 2003 (Non-patent document 8)) showed the importance of the structures of ceramides in the moisturizing and barrier functions of the skin, which raised questions about whether plant ceramides structurally different from human ceramides are highly functional lipids. Moreover, ceramides are present in animals and plants in minute amounts and are difficult to extract and purify, thus incurring low productivity and high cost, and therefore, it is highly desirable to develop a new production technique capable of overcoming these problems.

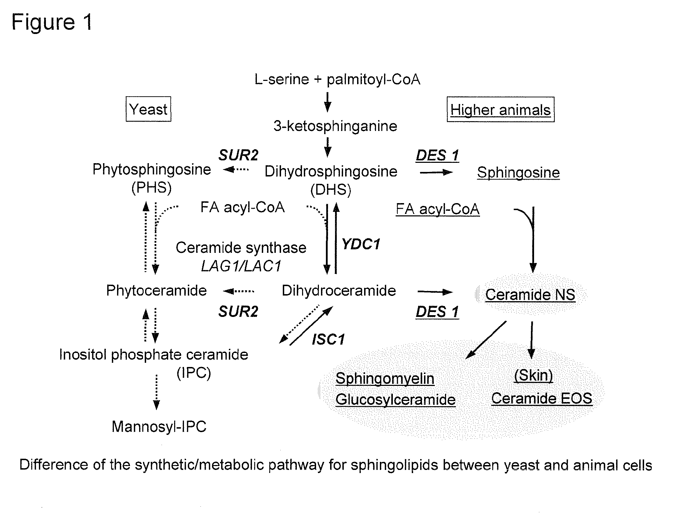

It is known that, in the synthetic/metabolic pathway for sphingolipids, reactions downstream of dihydrosphingosine (DHS) biosynthesis widely differ between higher animal cells (including human cells) and yeast cells, as shown in Figure 1. Each enzyme protein involved in various steps in the synthetic/metabolic pathway for sphingolipids and the gene encoding the protein have been known to some degree (Biochemistry. 41:15105-15114, 2002 (Non-patent document 9); J Biol Chem. 277:25512-25518, 2002 (Non-patent document 10); Yeast 9: 267-277, 1993 (Non-patent document 11); J Biol Chem 272:29704-29710, 1997 (Non-patent document 12); J Biol Chem 275:31369-31378, 2000 (Non-patent document 13); J Biol Chem 275:39793-39798, 2000 (Non-patent document 14)).
Non-patent document 1: J Invest Dermatol. 96:523-526, 1991
Non-patent document 2: Arch Dermatol Res. 283:219-223, 1991
Non-patent document 3: J Dermatol Sci. 1:79-83, 1990
Non-patent document 4: Acta Derm Venereol. 74:337-340, 1994
Non-patent document 5: Contact Dermatitis. 45:280-285, 2001
Non-patent document 6: J Eur Acad Dermatol Venereol. 16:587-594, 2002
Non-patent document 7: Cell Signal 14:779-785, 2002
Non-patent document 8: J. Clin. Invest. 112:1372-1382, 2003
Non-patent document 9: Biochemistry. 41:15105-15114, 2002
Non-patent document 10: J Biol Chem. 277:25512-25518, 2002
Non-patent document 11: Yeast 9: 267-277, 1993
Non-patent document 12: J Biol Chem 272:29704-29710, 1997
Non-patent document 13: J Biol Chem 275:31369-31378, 2000
Non-patent document 14: J Biol Chem 275:39793-39798, 2000
Non-patent document 15: EMBO J. 9:3153-3162, 1990
Non-patent document 16: J Cell Biol. 127:653-665, 1994
Non-patent document 17: Science. 263:1629-1631, 1994
Non-patent document 18: Cell. 79:1199-1207, 1994
Non-patent document 19: Eur J Cell Biol. 64:211-216, 1994
Non-patent document 20: Annu Rev Cell Dev Biol. 12:27-54, 1996
Non-patent document 21: J Cell Biol. 127:21-28, 1994
Non-patent document 22: J Biol Chem. 273:33273-33278, 1998
Non-patent document 23: J Cell Biol. 152:935-944, 2001

### DISCLOSURE OF THE INVENTION

### PROBLEMS TO BE SOLVED BY THE INVENTION

Plant ceramides structurally differ from human ceramide, and their productivity is low. To overcome these problems, it is highly desirable to develop a new production technique capable of producing human ceramide by using cells such as yeast cells.

### MEANS FOR SOLVING THE PROBLEMS

As a result of careful studies to solve the problems described above, the inventors of the present invention have achieved the present invention.

The present invention aims to develop a system for efficient production of highly functional human ceramide by gene recombination technology and successful control of the ceramide synthetic/metabolic and transport systems in eukaryotic organisms. To this end, budding yeast is used as a host because it is relatively easy to handle in gene manipulation and has been traditionally used for food manufacturing.

Specifically, a target of the present invention is ceramide NS, which exists in human stratum corneum with the highest distribution and is deemed crucial for the moisturizing and barrier functions of the skin. The structures of ceramides depend on the types of enzymes possessed by cells, and vary between species. Budding yeast used as a host possesses no enzyme for synthesizing ceramide NS, which is a main ceramide in higher animals. Instead, the host possesses an enzyme for synthesizing its inherent ceramide. Thus, to synthesize ceramide NS in yeast cells, the host-derived ceramide synthetic pathway must be inhibited and necessary enzymes must be introduced into yeast cells.

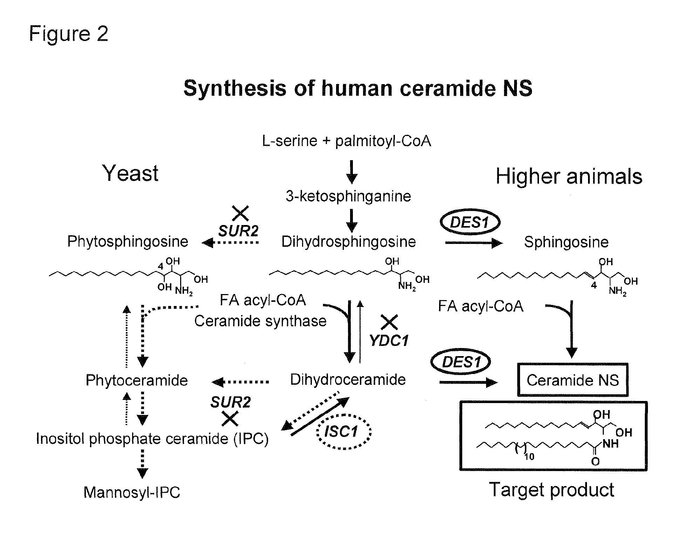

Specifically, in the synthetic/metabolic pathway for sphingolipids, reactions downstream of dihydrosphingosine (DHS) biosynthesis widely differ between higher animal cells (including human cells) and yeast cells, as shown in Figure 1. Namely, no human sphingoid base (sphingosine) having a double bond at C-4 of DHS is synthesized in budding yeast (genus Saccharomyces) due to the absence of the sphingolipid Δ4-desaturase gene (DES1) (Figure 2). Instead, phytosphingosine (PHS) is synthesized by hydroxylation of C-4 of DHS by an enzyme encoded by the sphinganine C4-hydroxylase gene (SUR2). Then, these sphingoid bases are converted into ceramides.

First, the inventors of the present invention thought it important to allow yeast cells to express a sphingolipid Δ4-desaturase enzyme not present in yeast cells and to completely or even partially abolish sphinganine C4-hydroxylase enzyme activity in order to produce human ceramide in yeast cells. Thus, the inventors initially attempted to 1) prepare the SUR2 gene disruption strain, and 2) introduce the human DES1 gene into the variant strain by transformation of yeast.

Further, once the introduced enzyme is localized at an optimum site, the amount of produced ceramide NS can be increased. Dihydroceramide, which is required for synthesis of ceramide NS, is synthesized in the endoplasmic reticulum of yeast. However, upon reviewing the C-terminal amino acid sequence of human sphingolipid Δ4-desaturase DES1 protein, there are no sequences for typical endoplasmic reticulum localization signals of yeast, for example, no dilysine-based sequences (KKXX or KXKXX). Thus, even when the human sphingolipid Δ4-desaturase DES1 protein is expressed in yeast cells, human ceramide NS cannot be synthesized efficiently.

The inventors of the present invention considered that for more efficient production of human ceramide, it would be more effective to allow a sphingolipid Δ4-desaturase DES1 protein serving as an enzyme to be localized within the endoplasmic reticulum. Namely, for localization of the DES1 protein within the endoplasmic reticulum, the sphingolipid Δ4-desaturase gene is modified to encode a typical yeast endoplasmic reticulum localization signal attached to the DES1 protein and the thus modified sphingolipid Δ4-desaturase gene (DES1m) is introduced into yeast cells. Once the introduced enzyme is localized at an optimum site, the amount of produced ceramide NS can be increased.

Accordingly, the present invention relates to a method for producing human ceramide in a yeast cell, which comprises:
1) introducing, by transformation of the yeast cell, an endoplasmic reticulum localization signal-attached sphingolipid Δ4-desaturase gene (DES1m) in which a nucleotide sequence encoding a yeast endoplasmic reticulum localization signal is added to the 3'-end of the sphingolipid Δ4-desaturase gene (DES1); and
2) abolishing, by transformation of the yeast cell, the expression of the yeast sphinganine C4-hydroxylase gene (SUR2).

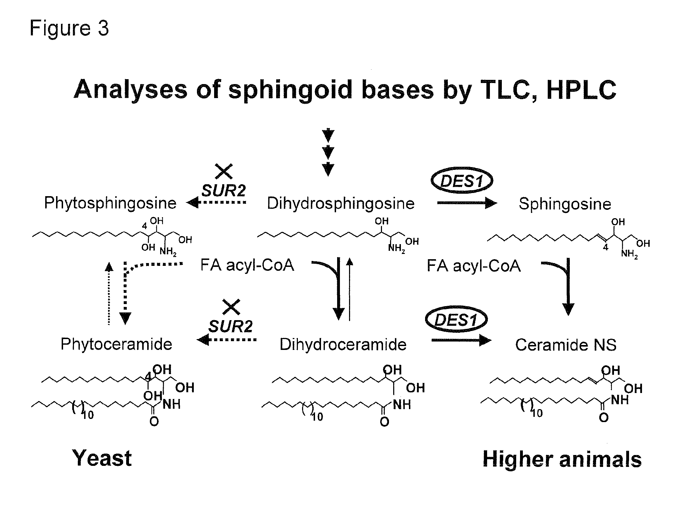

As used herein, human ceramide refers to ceramide NS having the structural formula shown as "target product" in Figure 2, for example. In contrast, phytoceramide is yeast ceramide, which differs from the human ceramide in that the double bond at the 4-position of the human ceramide is substituted by a hydroxy group (Figure 3).

The inventors further optimized the method of the present invention to construct a system for efficiently producing human ceramide NS. Specifically, the inventors succeeded in producing human ceramide more efficiently by transformation of yeast with any one of 3) or 4) shown below or a combination thereof:
3) abolishing the expression of the yeast sphingolipid α-hydroxylase gene (SCS7) to prevent hydroxylation of ceramide NS; and/or
4) abolishing the expression of the yeast alkaline dihydroceramidase gene (YDC1).

Thus, the present invention provides methods for conveniently and efficiently producing human ceramide in yeast cells by including 1) and 2) above as essential features, and 3) and/or 4) as additional features in preferred embodiments. The present invention preferably encompasses the following embodiments.

### (Embodiment 1)

A method for producing human ceramide in a yeast cell, which comprises:
1) introducing, by transformation of the yeast cell, an endoplasmic reticulum localization signal-attached sphingolipid Δ4-desaturase gene (DES1m) in which a nucleotide sequence encoding a yeast endoplasmic reticulum localization signal is added to the 3'-end of the sphingolipid Δ4-desaturase gene (DES1); and
2) abolishing, by transformation of the yeast cell, the expression of the yeast sphinganine C4-hydroxylase gene (SUR2).

### (Embodiment 2)

The method according to Embodiment 1, wherein the endoplasmic reticulum localization signal has an amino acid sequence composed of 4 or 5 amino acid residues including two or more lysine residues.

### (Embodiment 3)

The method according to Embodiment 1 or 2, wherein the endoplasmic reticulum localization signal has an amino acid sequence represented by VKKEK (wherein V denotes a valine residue, K denotes a lysine residue, and E denotes a glutamic acid).

### (Embodiment 4)

The method according to any one of Embodiments 1 to 3, wherein the sphingolipid Δ4-desaturase gene (DES1) encodes a protein having the amino acid sequence of SEQ ID NO: 2 or an amino acid sequence obtained by deletion, addition or substitution of one or more amino acid residues in SEQ ID NO: 2, and having sphingolipid Δ4-desaturase activity.

### (Embodiment 5)

The method according to any one of Embodiments 1 to 4, wherein the endoplasmic reticulum localization signal-attached sphingolipid Δ4-desaturase gene (DES1m) encodes a protein consisting of the amino acid sequence of SEQ ID NO: 4.

### (Embodiment 6)

The method according to any one of Embodiments 1 to 5, wherein the yeast sphinganine C4-hydroxylase gene (SUR2) encodes a protein having the amino acid sequence of SEQ ID NO: 6 or an amino acid sequence obtained by deletion, addition or substitution of one or more amino acid residues in SEQ ID NO: 6, and having sphinganine C4-hydroxylase activity.

### (Embodiment 7)

The method according to any one of Embodiments 1 to 6, which further comprises abolishing, by transformation of the yeast cell, the expression of the yeast sphingolipid α-hydroxylase gene (SCS7).

### (Embodiment 8)

The method according to Embodiment 7, wherein the yeast sphingolipid α-hydroxylase gene (SCS7) encodes a protein having the amino acid sequence of SEQ ID NO: 8 or an amino acid sequence obtained by deletion, addition or substitution of one or more amino acid residues in SEQ ID NO: 8, and having sphingolipid α-hydroxylase activity.

### (Embodiment 9)

The method according to any one of Embodiments 1 to 8, which further comprises abolishing, by transformation of the yeast cell, the expression of the yeast alkaline dihydroceramidase gene (YDC1).

### (Embodiment 10)

The method according to Embodiment 9, wherein the yeast alkaline dihydroceramidase gene (YDC1) encodes a protein having the amino acid sequence of SEQ ID NO: 10 or an amino acid sequence obtained by deletion, addition or substitution of one or more amino acid residues in SEQ ID NO: 10, and having alkaline dihydroceramidase activity.

### (Embodiment 11)

The method according to any one of Embodiments 1 to 10, wherein the yeast is selected from yeast species of the genus Saccharomyces.

### Sphingolipid Δ4-desaturase gene (DES1)

As essential feature 1), the methods of the present invention comprise introducing, by transformation of the yeast cell, an endoplasmic reticulum localization signal-attached sphingolipid Δ4-desaturase gene (DES1m) in which a nucleotide sequence encoding a yeast endoplasmic reticulum localization signal is added to the 3'-end of the sphingolipid Δ4-desaturase gene (DES1).

DES1 preferably encodes, but is not limited to, a protein having the amino acid sequence of SEQ ID NO: 2 or an amino acid sequence obtained by deletion, addition or substitution of one or more amino acid residues in SEQ ID NO: 2, and having sphingolipid Δ4-desaturase activity.

Genes (nucleic acids) that can be used in the present invention include genomic DNAs (including their corresponding cDNAs), chemically synthesized DNAs, DNAs amplified by PCR, and combinations thereof.

DES1 preferably has the nucleotide sequence of SEQ ID NO: 1. This is a nucleotide sequence encoding a human sphingolipid Δ4-desaturase protein having the amino acid sequence of SEQ ID NO: 2, and it is disclosed in, for example, GenBanK™: accession number AF466375.

One or more codons may encode the same amino acid, and this is called degeneracy of the genetic code. Thus, a DNA sequence not completely identical to SEQ ID NO: 1 may encode a protein having an amino acid sequence completely identical to SEQ ID NO: 2. Such a variant DNA sequence may result from silent mutation (e.g., occurring during PCR amplification), or can be the product of deliberate mutagenesis of a native sequence.

DES1 preferably encodes the amino acid sequence of SEQ ID NO: 2. However, it may also have an amino acid sequence obtained by deletion, addition or substitution of one or more amino acid residues. It is intended to encompass any homologous protein so long as it has sphingolipid Δ4-desaturase activity. The present invention is not limited to SEQ ID NO: 2 in so far as an amino acid sequence having a comparable function to that of SEQ ID NO: 2 is encoded. "Amino acid change" involves one or more amino acids, preferably 1-20, more preferably 1-10, most preferably 1-5 amino acids.

The amino acid sequence encoded by DES1 has an identity of at least about 70%, preferably about 80% or more, more preferably 90% or more, still more preferably 95% or more, and most preferably 98% or more to the amino acid sequence of SEQ ID NO: 2.

The percent amino acid identity may be determined by visual inspection and mathematical calculation. Alternatively, the percent identity of two protein sequences can be determined by comparing sequence information using the GAP computer program, based on the algorithm of Needleman, S.B. and Wunsch, C.D. (J. Mol. Biol., 48: 443-453, 1970), available from the University of Wisconsin Genetics Computer Group (UWGCG). The preferred default parameters for the GAP program include: (1) a scoring matrix, blosum62, as described by Henikoff, S and Henikoff, J.G. (Proc. Natl. Acad. Sci. USA, 89: 10915-10919, 1992); (2) a gap weight of 12; (3) a gap length weight of 4; and (4) no penalty for end gaps.

Other programs used by those skilled in the art of sequence comparison may also be used. The percent identity can be determined by comparing sequence information using the BLAST program described by Altschul et al. (Nucl. Acids. Res. 25, pp. 3389-3402, 1997), for example. This program is available at the website of National Center for Biotechnology Information (NCBI) or DNA Data Bank of Japan (DDBJ) on the Internet. Various conditions (parameters) for homology searches with the BLAST program are described in detail on the site, and searches are normally performed with default values, though some settings may be appropriately changed.

In the methods of the present invention, sphingolipid Δ4-desaturase preferably has the amino acid sequence of SEQ ID NO: 2 or an amino acid sequence at least 70% identical to SEQ ID NO: 2, and has sphingolipid Δ4-desaturase activity.

It is well known to those skilled in the art that even proteins having the same function may have different amino acid sequences depending on the varieties from which they are derived. DES1 may include such homologs and variants of the nucleotide sequence of SEQ ID NO: 1 so long as they have sphingolipid Δ4-desaturase activity. In addition to the human sphingolipid Δ4-desaturase protein of SEQ ID NO: 2, the presence of genes encoding proteins showing a similar activity is known in, for example, mouse (*M*. *musculus*), drosophila (*D. melanogaster*), nematode (*C*. *elegans*), fission yeast (*Schizosaccharomyces pombe*), etc. (Non-patent document 10).

The expression "having sphingolipid Δ4-desaturase activity" refers to the activity of introducing a double bond into C-4 of dihydrosphingosine to synthesize sphingosine, as shown in Figure 2 or Figure 3. Alternatively, it refers to the activity of introducing a double bond into C-4 of dihydroceramide to synthesize ceramide NS. Introduction of DES1 allows transformant yeast cells to synthesize sphingosine and/or ceramide NS that are not synthesized in the natural metabolic pathway of yeast.

A preferred sphingolipid Δ4-desaturase gene of the present invention also includes a nucleic acid capable of hybridizing to the nucleotide sequence of SEQ ID NO: 1 under stringent conditions, e.g., under conditions of moderate or high stringency and having sphingoid Δ4-desaturase activity.

The expression "under stringent conditions" refers to hybridization under conditions of moderate or high stringency. Specifically, conditions of moderate stringency can be readily determined by those having ordinary skill in the art based on, for example, the length of the DNA. The basic conditions are set forth by Sambrook et al., Molecular Cloning: A Laboratory Manual, 3rd Ed., Chapters 6-7, Cold Spring Harbor Laboratory Press, 2001, and include use of a prewashing solution for the nitrocellulose filters 5 x SSC, 0.5% SDS, 1.0 mM EDTA (pH 8.0), hybridization conditions of about 50% formamide, 2 x SSC to 6 x SSC at about 40°C to 50°C (or other similar hybridization solution, such as Stark's solution, in about 50% formamide at about 42°C), and washing conditions of about 40°C to 60°C, 0.5 to 6 x SSC, 0.1% SDS. Preferably, conditions of moderate stringency include hybridization conditions (and washing conditions) of 6 x SSC at about 50°C. Conditions of high stringency can also be readily determined by the skilled artisan based on, for example, the length of the DNA.

Generally, such conditions include hybridization and/or washing at higher temperatures and/or lower salt concentrations than in the conditions of moderate stringency (e.g., hybridization in 6 x SSC to 0.2 x SSC, preferably 6 x SSC, more preferably 2 x SSC, most preferably 0.2 x SSC at about 65°C), and are defined to involve hybridization conditions as above and washing in 0.2 x SSC, 0.1% SDS at about 65°C to 68°C. SSPE (1 x SSPE = 0.15 M NaCl, 10 mM NaH₂PO₄, and 1.25 mM EDTA, pH 7.4) can be substituted for SSC (1 x SSC = 0.15 M NaCl and 15 mM sodium citrate) for use as hybridization and washing buffers, and washing is continued for 15 minutes after completion of hybridization.

As known by those skilled in the art and as further described below, it should be understood that the washing temperature and the washing salt concentration can be adjusted as desired to achieve a desirable degree of stringency by applying basic principles governing hybridization reaction and duplex stability (see, e.g., Sambrook et al., 2001). When a nucleic acid is to be hybridized to a target nucleic acid of an unknown sequence, the length of the hybrid is assumed to be that of the nucleic acid to be hybridized. When nucleic acids of known sequences are to be hybridized, the length of the hybrid can be determined by aligning the sequences of the nucleic acids and identifying a single or multiple region(s) having optimal sequence complementarity. The hybridization temperature of a hybrid estimated to have a length of less than 50 bp must be 5-25°C lower than the melting temperature (Tₘ) of the hybrid, where Tₘ is determined by the equation below. For hybrids having a length of less than 18 bp, Tₘ (°C) = 2 (the number of A+T bases) + 4 (the number of G+C bases). For hybrids having a length of 18 bp or more, Tₘ = 81.5°C + 16.6 (log₁₀[Na⁺]) + 41 (mole fraction [G+C]) - 0.63 (% formamide) - 500/n, where N is the number of bases in the hybrid, and [Na⁺] is the sodium ion concentration in the hybridization buffer ([Na⁺] in 1 x SSC = 0.165 M). Preferably, such hybridizing nucleic acids each have a length of at least 8 nucleotides (or more preferably at least 15 nucleotides, or at least 20 nucleotides, or at least 25 nucleotides, or at least 30 nucleotides, or at least 40 nucleotides, or most preferably at least 50 nucleotides), or a length of at least 1% (more preferably at least 25%, or at least 50%, or at least 70%, and most preferably at least 80%) of the length of a nucleic acid to which it hybridizes, and has a sequence identity of at least 50% (more preferably at least 70%, at least 75%, at least 80%, at least 85%, at least 90%, at least 95%, at least 97.5%, or at least 99%, and most preferably at least 99.5%) to a nucleic acid to which it hybridizes. The sequence identity here is determined by comparing the sequences of the nucleic acids to be hybridized when aligned so as to maximize overlap and identity while minimizing sequence gaps, as described in detail above.

The percent nucleic acid identity can be determined by visual inspection and mathematical calculation. Alternatively, the percent identity of two nucleic acid sequences can be determined by visual inspection and mathematical calculation, or more preferably, the comparison is made by comparing sequence information using a computer program. An exemplary, preferred computer program is the Genetics Computer Group (GCG; Madison, Wis.) Wisconsin package version 10.0 program, "GAP" (Devereux et al., 1984, Nucl. Acids Res. 12: 387). This "GAP" program can be used to compare not only two nucleic acid sequences but also two amino acid sequences or a nucleic acid sequence and an amino acid sequence. The preferred default parameters for the "GAP" program include (1) The GCG implementation of a unary comparison matrix (containing a value of 1 for identities and 0 for non-identities) for nucleotides, and the weighted amino acid comparison matrix of Gribskov and Burgess, Nucl. Acids Res. 14: 6745, 1986 as described by Schwartz and Dayhoff, eds., "Atlas of Polypeptide Sequence and Structure", National Biomedical Research Foundation, pp. 353-358, 1979; or other comparable comparison matrices; (2) a penalty of 30 for each gap and an additional penalty of 1 for each symbol in each gap for amino acid sequences, or penalty of 50 for each gap and an additional penalty of 3 for each symbol in each gap for nucleotide sequences; (3) no penalty for end gaps; and (4) no maximum penalty for long gaps. Other programs used by those skilled in the art of sequence comparison can also be used, such as, for example, the BLASTN program version 2.2.7, available for use via the National Library of Medicine website: http://www.ncbi.nlm.nih.gov/blast/b12seq/hls.ht ml, or the WU-BLAST 2.0 algorithm. Standard default parameter settings for WU-BLAST 2.0 are described at the following Internet site: http://blast.wustl.edu. In addition, the BLAST algorithm uses the BLOSUM62 amino acid scoring matix, and optional parameters that can be used are as follows: (A) inclusion of a filter to mask segments of the query sequence that have low compositional complexity (as determined by the SEG program of Wootton and Federhen (Computers and Chemistry, 1993); also see Wootton and Federhen, 1996, Analysis of compositionally biased regions in sequence databases, Methods Enzymol. 266: 554-71) or segments consisting of short-periodicity internal repeats (as determined by the XNU program of Claverie and States (Computers and Chemistry, 1993)), and (B) a statistical significance threshold for reporting matches against database sequences, or E-score (the expected probability of matches being found merely by chance, according to the stochastic model of Karlin and Altschul, 1990; if the statistical significance ascribed to a match is greater than this E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The sphingolipid Δ4-desaturase gene (DES1) of the present invention also includes a nucleic acid having a nucleotide sequence different from that of SEQ ID NO: 1 by deletion, insertion or substitution of one or more nucleotides, but still encoding a protein having sphingolipid Δ4-desaturase activity. The number of nucleotides deleted, inserted or substituted is not limited so long as a protein having sphingolipid Δ4-desaturase activity is encoded, but is preferably 1 to several thousands, more preferably 1 to 1,000, more preferably 1 to 500, still more preferably 1 to 200, most preferably 1 to 100.

A given amino acid may be replaced, for example, by a residue having similar physiochemical characteristics. Examples of such conservative substitutions include changes from one aliphatic residue to another, such as changes from one to another of Ile, Val, Leu, or Ala; changes from one polar residue to another, such as Lys to Arg, Glu to Asp, or Gln to Asn; or changes from one aromatic residue to another, such as changes from one to another of Phe, Trp, or Tyr. Other well-known conservative substitutions include, for example, changes between entire regions having similar hydrophobic characteristics. Those skilled in the art can introduce desired deletions, insertions or substitutions by well-known gene engineering techniques using, for example, site-specific mutagenesis as described in Sambrook et al. (2001), supra.

### Endoplasmic reticulum localization signal-attached sphingolipid Δ4-desaturase gene (DES1m)

The endoplasmic reticulum localization signal-attached sphingolipid Δ4-desaturase gene (DES1m) of the present invention is a gene in which a nucleotide sequence encoding a yeast endoplasmic reticulum localization signal is added to the 3'-end of the sphingolipid Δ4-desaturase gene (DES1) described above. "Yeast endoplasmic reticulum localization signals" are well known to those skilled in the art, and any endoplasmic reticulum localization signal can be used in the methods of the present invention.

For example, without being limited thereto, the following documents disclose typical yeast localization signals, each having an amino acid sequence composed of 4 or 5 amino acid residues including two or more lysine residues.

EMBO J. 9:3153-3162, 1990 (Non-patent document 15)
J Cell Biol. 127:653-665, 1994 (Non-patent document 16)
Science. 263:1629-1631, 1994 (Non-patent document 17)
Cell. 79:1199-1207, 1994 (Non-patent document 18)
Eur J Cell Biol. 64:211-216, 1994 (Non-patent document 19)
Annu Rev Cell Dev Biol. 12:27-54, 1996 (Non-patent document 20)
The "yeast endoplasmic reticulum localization signal" intended in the present invention preferably "has an amino acid sequence composed of 4 or 5 amino acid residues including two or more lysine residues." More preferably, the endoplasmic reticulum localization signal has an amino acid sequence containing KKX₁X₂ or KX₁KX₂X₃ (wherein K denotes a lysine residue, and X₁, X₂ and X₃ represent any amino acid residues which are the same or different). As one embodiment, the amino acid sequence of the most preferred endoplasmic reticulum localization signal contains VKKEK (wherein V denotes a valine residue, K denotes a lysine residue, and E denotes a glutamic acid) described later in the EXAMPLE section.

In the endoplasmic reticulum localization signal-attached sphingolipid Δ4-desaturase gene (DES1m) of the present invention, the segment other than the signal, i.e., the sphingolipid Δ4-desaturase gene (DES1) is not limited in any way, and is as described above in the section "Sphingolipid Δ4-desaturase gene (DES1)." Preferably, it encodes the amino acid sequence of SEQ ID NO: 2. Thus, the DES1m gene encodes a protein consisting of the amino acid sequence of SEQ ID NO: 2 + VKKEK, i.e., consisting of the amino acid sequence of SEQ ID NO: 4. The nucleic acid sequence of the DES1m gene typically consists of the nucleotide sequence of SEQ ID NO: 3. In addition to SEQ ID NO: 3, other nucleotide sequences are encompassed so long as they encode the amino acid sequence of SEQ ID NO: 4 as a result of genetic degeneracy.

A wide range of "yeast endoplasmic reticulum localization signals" are known, in addition to those "having an amino acid sequence composed of 4 or 5 amino acid residues including two or more lysine residues." Without being limited thereto, for example, mechanisms of endoplasmic reticulum localization are known and can be used in the methods of the present invention.

J Cell Biol. 127:21-28, 1994 (Non-patent document 21)
This document shows that proteins containing the amino acid sequence HDEL are retrieved from the Golgi apparatus to the endoplasmic reticulum. As in the case of HDEL, KDEL is also known as an endoplasmic reticulum localization signal.

J Biol Chem. 273:33273-33278, 1998 (Non-patent document 22)
This document discloses that determinant(s) in transmembrane domains act on retrieval to the endoplasmic reticulum.

J Cell Biol. 152:935-944, 2001 (Non-patent document 23)
This document shows that a member of Golgi membrane proteins is involved in retrieval to the endoplasmic reticulum through interaction with a retrieval signal in the transmembrane domain of an endoplasmic reticulum membrane protein.

It should be noted that any cell other than a yeast cell can also be used as a host cell for transformation in the methods of the present invention. Examples of a host cell include mammalian cells (e.g., human, mouse and rat cells), insect cells, etc. In these various types of cells, signals for endoplasmic reticulum localization are known, and it is possible to use an endoplasmic reticulum localization signal suitable for the type of cell to be used.

### Yeast sphinganine C4-hydroxylase gene (SUR2)

As essential feature 2), the methods of the present invention comprise abolishing the expression of the yeast sphinganine C4-hydroxylase gene (SUR2) by transformation of the yeast cell.

SUR2 preferably encodes, but is not limited to, a protein having the amino acid sequence of SEQ ID NO: 6 or an amino acid sequence obtained by deletion, addition or substitution of one or more amino acid residues in SEQ ID NO: 6, and having sphinganine C4-hydroxylase activity.

SUR2 preferably has the nucleotide sequence of SEQ ID NO: 5. This is a nucleotide sequence encoding a yeast sphinganine C4-hydroxylase protein having the amino acid sequence of SEQ ID NO: 6, and it is disclosed in, for example, SGD (Saccharomyces Genome Database, http://www.yeastgenome.org/).

SUR2 preferably encodes the amino acid sequence of SEQ ID NO: 6. However, it may also have an amino acid sequence obtained by deletion, addition or substitution of one or more amino acid residues. It is intended to encompass any homologous protein so long as it has sphinganine C4-hydroxylase activity. The present invention is not limited to SEQ ID NO: 6 so long as an amino acid sequence having a comparable function to that of SEQ ID NO: 6 is encoded. "Amino acid change" involves one or more amino acids, preferably 1-20, more preferably 1-10, most preferably 1-5 amino acids.

The expression "having sphinganine C4-hydroxylase activity" refers to the activity of introducing a hydroxyl group into C-4 of dihydrosphingosine to synthesize phytosphingosine, as shown in Figure 2 or Figure 3. Alternatively, it refers to the activity of introducing a hydroxyl group into C-4 of dihydroceramide to synthesize phytoceramide. In the present invention, the synthesis of phytosphingosine and/or phytoceramide that are synthesized in the natural metabolic pathway of yeast is partially or completely inhibited by partially or completely abolishing the expression of SUR2 by transformation of the yeast cell. Sphingosine and/or ceramide NS can be efficiently synthesized by suppressing the expression of the SUR2 gene and expressing the DES1 gene.

The amino acid sequence encoded by SUR2 has an identity of at least about 70%, preferably about 80% or more, more preferably 90% or more, still more preferably 95% or more, most preferably 98% or more to the amino acid sequence of SEQ ID NO: 6.

A preferred yeast sphinganine C4-hydroxylase gene (SUR2) of the present invention also includes a nucleic acid capable of hybridizing to the nucleotide sequence of SEQ ID NO: 5 under stringent conditions, e.g., under conditions of moderate or high stringency and having yeast sphinganine C4-hydroxylase activity.

The yeast sphinganine C4-hydroxylase gene (SUR2) of the present invention also includes a nucleic acid having a nucleotide sequence different from that of SEQ ID NO: 5 by deletion, insertion or substitution of one or more nucleotides, but still encoding a protein having sphinganine C4-hydroxylase activity.

Common matters such as "deletion, addition or substitution of amino acids and/or nucleotides", "percent identity of amino acids and/or nucleotides", hybridization "under stringent conditions" are as described above for DES1.

### Yeast sphingolipid α-hydroxylase gene (SCS7)

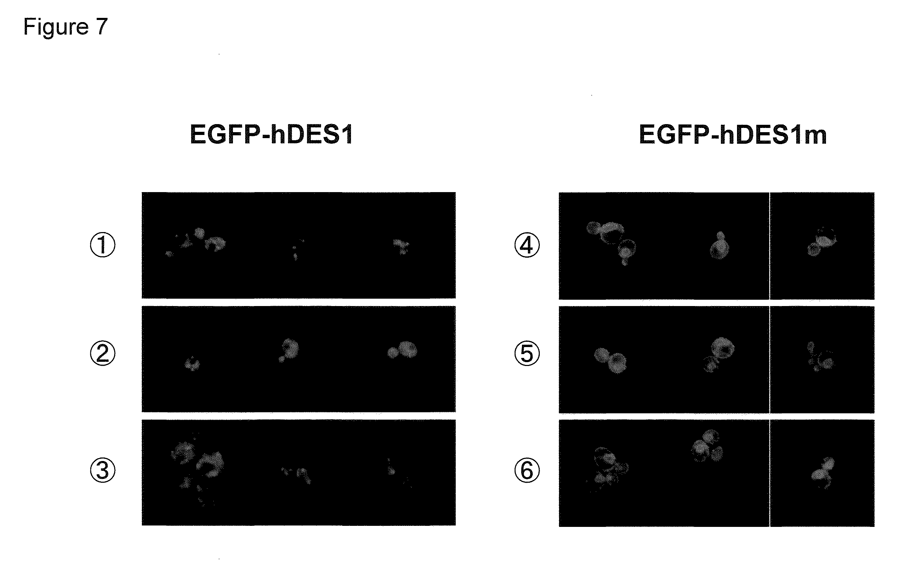

The methods of the present invention may further comprise abolishing the expression of the yeast sphingolipid α-hydroxylase gene (SCS7) by transformation of the yeast cell. SCS7 has the activity of adding a hydroxyl group to the α-carbon of a fatty acid amide-linked to the sphingoid base of phytoceramide, dihydroceramide, and ceramide NS to synthesize Cer(AP), Cer(ASa), and Cer(AS), respectively, in Figure 3 or Figure 7, for example. Even if desired dihydroceramide or ceramide NS is synthesized, it is further hydroxylated by the presence of SCS7. Thus, the present invention preferably comprises abolishing the expression of SCS7.

SCS7 preferably encodes, but is not limited to, a protein having the amino acid sequence of SEQ ID NO: 8 or an amino acid sequence obtained by deletion, addition or substitution of one or more amino acid residues in SEQ ID NO: 8, and having sphingolipid α-hydroxylase activity.

SCS7 preferably has the nucleotide sequence of SEQ ID NO: 7. This is a nucleotide sequence encoding a yeast sphingolipid α-hydroxylase protein having the amino acid sequence of SEQ ID NO: 8, and it is disclosed in, e.g., SGD (Saccharomyces Genome Database, http://www.yeastgenome.org/).

SCS7 preferably encodes the amino acid sequence of SEQ ID NO: 8. However, it may also have an amino acid sequence obtained by deletion, addition or substitution of one or more amino acid residues. It is intended to encompass any homologous protein so long as it has sphingolipid α-hydroxylase activity. The present invention is not limited to SEQ ID NO: 8 so long as an amino acid sequence having a comparable function to that of SEQ ID NO: 8 is encoded. "Amino acid change" involves one or more amino acids, preferably 1-20, more preferably 1-10, most preferably 1-5 amino acids.

The expression "having sphingolipid α-hydroxylase activity" refers to the activity of adding a hydroxyl group to the α-carbon of a fatty acid amide-linked to the sphingoid base of phytoceramide, dihydroceramide, and ceramide NS to synthesize Cer(AP), Cer(ASa), and Cer(AS), respectively, as shown in Figure 7, for example. In the present invention, undesirable hydroxylation of dihydroceramide or ceramide NS is inhibited by partially or completely abolishing the expression of SCS7, preferably by transformation of the yeast cell.

The amino acid sequence encoded by SCS7 has an identity of at least about 70%, preferably about 80% or more, more preferably 90% or more, still more preferably 95% or more, most preferably 98% or more to the amino acid sequence of SEQ ID NO: 8.

A preferred yeast sphingolipid α-hydroxylase gene (SCS7) of the present invention also includes a nucleic acid capable of hybridizing to the nucleotide sequence of SEQ ID NO: 7 under stringent conditions, e.g., under conditions of moderate or high stringency and having yeast sphingolipid α-hydroxylase activity.

The yeast sphingolipid α-hydroxylase gene (SCS7) of the present invention also includes a nucleic acid having a nucleotide sequence different from that of SEQ ID NO: 7 by deletion, insertion or substitution of one or more nucleotides, but still encoding a protein having sphingolipid α-hydroxylase activity.

Common matters such as "deletion, addition or substitution of amino acids and/or nucleotides", "percent identity of amino acids and/or nucleotides", hybridization "under stringent conditions" are as described above for DES1.

### Yeast alkaline dihydroceramidase gene (YDC1)

The methods of the present invention may further comprise abolishing the expression of the yeast alkaline dihydroceramidase gene (YDC1) by transformation of the yeast cell.

YDC1 encodes a protein having the activity of degrading dihydroceramide to synthesize dihydrosphingosine, as shown in Figure 1 to Figure 3, for example. YDC1 activity promotes so to speak a reverse reaction to ceramide synthesis to reduce dihydroceramide as a precursor for ceramide NS synthesis. Thus, the present invention preferably comprises abolishing the expression of YDC1.

YDC1 preferably encodes, but is not limited to, a protein having the amino acid sequence of SEQ ID NO: 10 or an amino acid sequence obtained by deletion, addition or substitution of one or more amino acid residues in SEQ ID NO: 10, and having alkaline dihydroceramidase activity.

YDC1 preferably has the nucleotide sequence of SEQ ID NO: 9. This is a nucleotide sequence encoding a yeast alkaline dihydroceramidase protein having the amino acid sequence of SEQ ID NO: 10, and it is disclosed in, for example, SGD (Saccharomyces Genome Database, http://www.yeastgenome.org/).

YDC1 preferably encodes the amino acid sequence of SEQ ID NO: 10. However, it may also have an amino acid sequence obtained by deletion, addition or substitution of one or more amino acid residues. It is intended to encompass any homologous protein so long as it has alkaline dihydroceramidase activity. The present invention is not limited to SEQ ID NO: 10 so long as an amino acid sequence having a comparable function to that of SEQ ID NO: 10 is encoded. "Amino acid change" involves one or more amino acids, preferably 1-20, more preferably 1-10, and most preferably 1-5 amino acids.

The expression "having alkaline dihydroceramidase activity" refers to the activity of degrading dihydroceramide to synthesize dihydrosphingosine, as shown in Figure 1 to Figure 3, for example. In the present invention, undesirable degradation of dihydroceramide is inhibited by partially or completely abolishing the expression of YDC1, preferably by transformation of the yeast cell.

The amino acid sequence encoded by YDC1 has an identity of at least about 70%, preferably about 80% or more, more preferably 90% or more, still more preferably 95% or more, most preferably 98% or more to the amino acid sequence of SEQ ID NO: 10.

A preferred yeast alkaline dihydroceramidase gene (YDC1) of the present invention also includes a nucleic acid capable of hybridizing to the nucleotide sequence of SEQ ID NO: 9 under stringent conditions, e.g., under conditions of moderate or high stringency and having yeast alkaline dihydroceramidase activity.

The yeast alkaline dihydroceramidase gene (YDC1) of the present invention also includes a nucleic acid having a nucleotide sequence different from that of SEQ ID NO: 9 by deletion, insertion or substitution of one or more nucleotides, but still encoding a protein having alkaline dihydroceramidase activity.

Common matters such as "deletion, addition or substitution of amino acids and/or nucleotides", "percent identity of amino acids and/or nucleotides", hybridization "under stringent conditions" are as described above for DES 1.

### Methods for introducing and expressing genes by transformation of yeast.

In the present invention, the expression of DES1m in the yeast cell can be performed by any known method. Preferably, the method comprises transforming a host yeast cell with an expression vector containing DES1m, and cultivating the transformant yeast cell under conditions allowing the expression of the nucleic acid.

The yeast species that can be used in the methods of the present invention are preferably, but are not limited to, yeast species of the genus Saccharomyces. *Saccharomyces cerevisiae, Saccharomyces pastorianus, Saccharomyces bayanus,* and *Saccharomyces kluyveri* are more preferred. Budding yeast species including the genus Saccharomyces have been analyzed most extensively for ceramide synthesis and metabolism at the genetic level. Thus, they can be used to rapidly optimize the methods for producing human ceramide according to the present invention. Moreover, yeast cells are easy to culture and have been traditionally used for food manufacturing. In addition, they can be used to establish a method for extracting/purifying large amounts of ceramides conveniently, safely and inexpensively.

In the present invention, known yeast expression vectors can be used to introduce and express genes. In the examples below, known gene expression vectors for yeast pRS series (p4XX) (Mumberg et al., Gene, 156, 119, 1995) and pYE22m (Ashikari et al., Appl Microbiol Biotechnol, 30, 515, 1989) were used.

Any of multicopy (YEp), single copy (YCp), and chromosome integration (YIp) vectors can be used for introduction into yeast. For example, a number of expression vectors for yeast are known to those skilled in the art and can be used in the methods of the present invention, including YEp vectors such as YEp24 (J. R. Broach et al., Experimental Manipulation of Gene Expression, Academic Press, New York, 83, 1983), YCp vectors such as YCp50 (M. D. Rose et al., gene, 60, 237, 1987), and YIp vectors such as YIp5 (K. Struhl et al., Proc. Natl. Acad. Sci. USA, 76, 1035, 1979).

In addition to each gene of interest, expression vectors can typically contain a selectable marker and an origin of replication for proliferation in host cells. Vectors also optionally contain a transcription or translation regulatory sequence preferably derived from yeast fused to a nucleic acid of the present invention.

Examples of regulatory sequences include transcriptional promoters, operators, or enhancers, an mRNA ribosomal binding site, and appropriate sequences which control the initiation and termination of transcription and translation. Nucleotide sequences are operably linked to a regulatory sequence when the regulatory sequence is functionally associated with the DNA sequences. Thus, a promoter nucleotide sequence is operably linked to a DNA sequence if the promoter nucleotide sequence controls the transcription of the DNA sequence. An origin of replication that confers the ability to replicate in a host cell, and a selection gene by which transformants are identified are generally incorporated into expression vectors. As for selectable markers, those commonly used can be routinely used. Examples are genes resistant to antibiotics such as tetracycline, ampicillin, kanamycin, neomycin, hygromycin or spectinomycin and auxotrophic genes such as HIS3, TRI.

Yeast vectors will often contain an origin of replication sequence derived from the 2µ yeast plasmid, an autonomous replication sequence (ARS), a promoter region, a sequence for polyadenylation, a sequence for transcription termination, and a selectable marker gene.

Vectors can be conveniently prepared by routine fusion of a desired gene to a recombination vector available in the art (e.g., plasmid DNA). Methods for integrating a DNA fragment of a gene into a vector such as a plasmid are described in, for example, Sambrook, J., and Russell, D.W. (2001). Molecular Cloning: A Laboratory Manual, 3rd ed. (New York: Cold Spring Harbor Laboratory Press). Commercially available ligation kits (e.g., available from TAKARA) can be conveniently used.

Methods for introducing a vector into a host cell include calcium phosphate or calcium chloride/rubidium chloride transfection, electroporation, electroinjection, chemical treatment with PEG or the like, the use of a gene gun, etc. described in Sambrook, J. et al. (2001) (supra.).

### Methods for abolishing each gene by transformation of yeast.

The methods of the present invention comprise abolishing the expression of the yeast sphinganine C4-hydroxylase gene (SUR2) by transformation of the yeast cell.

In preferred embodiments, the methods of the present invention further comprise either one or a combination of:
abolishing the expression of the yeast sphingolipid α-hydroxylase gene (SCS7) by transformation of the yeast cell; and
abolishing the expression of the yeast alkaline dihydroceramidase gene (YDC1) by transformation of the yeast cell.

As used herein, the expression "abolishing the expression of each gene" means that the protein activity encoded by each gene is not produced. Any abolishment is included in the scope of the present invention to achieve the purposes in the methods of the present invention, so long as the protein activity encoded by the gene is not exerted eventually, such as disrupting the gene on the genome of a yeast cell, inhibiting the transcription of the gene, inhibiting the translation of the gene into a protein, or inhibiting activity even if it is translated into a protein. Abolishment may be partial or complete. Typically, each gene on the genome of a mother cell is disrupted to partially or completely delete the gene.

The expression of the SUR2, SCS7 and YDC1 genes can be abolished by known methods.

For example, a DNA fragment containing upstream and downstream nucleotide sequences of each gene fused to a selectable marker was used to delete the gene by homologous recombination with the natural genome sequence of yeast in the examples herein below.

Disruption of a gene can be performed by addition or deletion of one or more nucleotides in a region responsible for the expression of a gene product in the target gene, such as a coding region or a promoter region, or by entire deletion of these regions. Such methods for gene disruption can be found in known publications (e.g., see Yeast 10, 1793 (1994), Yeast 15, 1541 (1999), Proc. Natl. Acad. Sci. USA, 76, 4951 (1979), Methods in Enzymology, 101, 202 (1983), etc.).

In addition to gene disruption, other methods for suppressing the expression of each gene for the purposes of the present invention include antisense methods (e.g., see Hirajima and Inoue: New Biochemistry Experiment Course 2 Nucleic acid, IV. Gene Replication and Expression (Japanese Biochemical Society Ed., Tokyo Kagaku Dozin Co., Ltd.) pp. 319-347, 1993, etc.), RNAi methods (see Domestic announcement No. 2002-516062 of PCT application; US Patent Laid-Open Publication No. 2002/086356A; Nature Genetics, 24 (2), 180-183, 2000, etc.), ribozyme methods (see FEBS Lett. 228: 228, 1988; FEBS Lett. 239: 285, 1988; Nucl. Acids. Res. 17: 7059, 1989, etc.), cosuppression (e.g., see Smyth DR: Curr. Biol. 7: R793, 1997, Martienssen R: Curr. Biol. 6: 810, 1996, etc.), etc.

### Methods for verifying ceramide synthesis

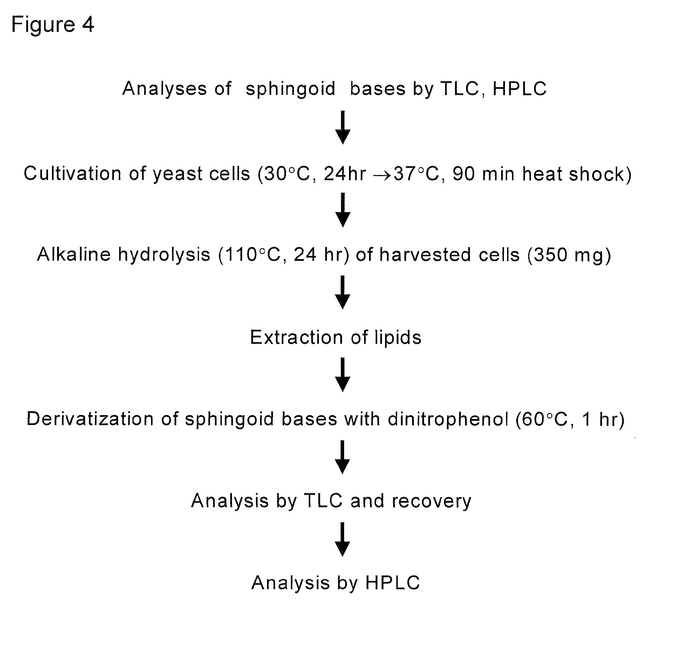

The human ceramide (ceramide NS) produced by the methods of the present invention can be extracted/purified by using known methods. The methods of the present invention allow large-scale culture and convenient and rapid extraction/purification of the ceramide because yeast cells are used. The purified ceramide can be identified by using known methods for analyzing sphingoid bases. Analytical methods include, e.g., TLC and HPLC as shown in Figure 4, mass spectrometry (e.g., LC-MS, LC-MS/MS, FT-MS), etc.

### ADVANTAGES OF THE INVENTION

When using the system of the present invention for efficient production of ceramide NS by enzyme localization control, human ceramide highly functional on the human skin can be produced more inexpensively.

### BRIEF DESCRIPTION OF THE DRAWINGS

[Figure 1] Figure 1 shows the synthetic/metabolic pathways for sphingolipids in yeast and higher animal cells.
[Figure 2] Figure 2 shows an overview of a preferred embodiment of a method for producing human ceramide in yeast cells according to the present invention.
[Figure 3] Figure 3 shows the molecular species of sphingoid bases and ceramides and the structural formulae thereof in yeast and higher animals.
[Figure 4] Figure 4 schematically shows steps from cultivation of yeast cells to analyses by TLC and HPLC.
[Figure 5] Figure 5 shows the results of TLC analysis of yeast ceramides using tritiated (³H) D-erythro-dihydrosphingosine and the results of quantification of radioactively labeled ceramides using a Bioimage Analyzer (BAS). Tritiation was performed for 16 hours at a temperature of 25°C. From the left, Samples 1-3 are as follows.

1. Example 6III. SUR2/SCS7 double disruption (Example 3) + empty vector
2. Example 6I. SUR2/SCS7 double disruption (Example 3) + DES1 gene expression
3. Example 6II. SUR2/SCS7 double disruption (Example 3) + DES1m gene expression
[Figure 6] Figure 6 shows the results of TLC analysis of yeast ceramides using tritiated (³H) D-erythro-dihydrosphingosine and the results of quantification of radioactively labeled ceramides using a Bioimage Analyzer (BAS). Tritiation was performed for 16 hours at a temperature of 25°C. From the left, Samples 1-3 are as follows.

1. Example 6VI. SUR2/SCS7/YDC1 triple disruption (Example 4) + empty vector
2. Example 6IV SUR2/SCS7/YDC1 triple disruption (Example 4) + DES1 gene expression
3. Example 6V SUR2/SCS7/YDC1 triple disruption (Example 4) + DES1m gene expression
[Figure 7] Figure 7 shows the results observed for localization of the fusion proteins prepared in Example 9 under a fluorescent microscope (OLMPUS BX51) with a GFP filter.

### EXAMPLES

The following examples further illustrate the present invention but are not intended to limit the technical scope of the invention. Those skilled in the art can readily add modifications/changes to the present invention in the light of the description herein, and those modifications/changes are included in the technical scope of the present invention.

### Example 1: Preparation of expression vector for human sphingolipid Δ4-desaturase gene (DES1)

Based on the nucleotide sequence of the human sphingolipid Δ4-desaturase gene (DES1) (SEQ ID NO: 40, CDS is shown in SEQ ID NO: 1) in a public database (GenBanK™: accession number AF466375), primers des1f (SEQ ID NO: 11) and des1R (SEQ ID NO: 12) were prepared.

SEQ ID NO: 11:
   5'-CCTTCTCTAGAGGATCCATGGGGAGCCGCGTCTCGCGGGAAGAC-3'
SEQ ID NO: 12:
   5'-CCTTCGAATTCCCCGGGCCAGGGGAGCTTCTGAGCATCACTGGTC-3'
The primer pair was used to perform PCR with a human cDNA library as a template. The resulting PCR product (about 1.1 kb) was cloned into the gene expression vector for yeast pKO11 (Kamei et al., J. Biol. Chem., 273, 28341, 1998; provided by Dr. K. Tanaka) using BamHI and SmaI sites.

The nucleotide sequence of the clone was determined by the Sanger method to confirm that it was identical to the sequence in the database. The clone was subcloned into the gene expression vector for yeast pRS series (p4XX) (Mumberg et al., Gene, 156, 119, 1995) using BamHI and Xho1 sites (phDES1).

### Example 2: Preparation of disruption strain of yeast sphinganine C4-hydroxylase gene (SUR2)

Based on a sequence (SEQ ID NO: 41) containing the nucleotide sequence of the yeast sphinganine C4-hydroxylase gene (SUR2) (SEQ ID NO: 5) and its upstream and downstream regions in a public yeast genome database (SGD (Saccharomyces Genome Database, http://www.yeastgenome.org/)), primers sur2F (SEQ ID NO: 13) and sur2R (SEQ ID NO: 14) were prepared.

SEQ ID NO: 13:
5'-CTCCGGCTTCTGCGGTTTTTCTTAGTCTTTCCGCACCAATTTTCACAGGAATTC
CCGGGGATCCGG-3'

SEQ ID NO: 14:
5'-GGATAATAAATACAAACGTGGGAAGTCGGAGACATTGCCTTTACCCAGCAAGC
TAGCTTGGCTGCAGG-3'

The primer pair was used to perform PCR with plasmid pYDp-L (Berben et al., Yeast, 7, 475, 1991) as a template, thereby giving a PCR product containing a 295-bp upstream region of the SUR2 gene, a selectable marker and a 75-bp downstream region of the SUR2 gene fused together. This PCR product was routinely transformed into the strain FK113 (MATa, ura3, his3, leu2, lys2, trp1, bar1-1), and transformants were selected in an auxotrophic medium to give a SUR2 gene disruption strain.

The disruption of the SUR2 gene was confirmed by PCR using confirmation primers designed to be amplified into fragments of different lengths depending on whether the gene is normal or disrupted (SEQ ID NOs: 15 and 16).

SEQ ID NO: 15: 5'-CTCCGGCTTCTGCGGTTTTTCTTAGTCTTTC-3'
SEQ ID NO: 16: 5'-GGAAGTCGGAGACATTGCCTTTACCCAG-3'

### Example 3: Preparation of double disruption strain of yeast SUR2 and yeast sphingolipid α-hydroxylase SCS7) genes

Based on a sequence (SEQ ID NO: 42) containing the nucleotide sequence of the yeast sphingolipid α-hydroxylase gene (SCS7) (SEQ ID NO: 7) and its upstream and downstream regions in a public yeast genome database (SGD), primers scs7up280F (SEQ ID NO: 17) and scs7up280R_G418 (SEQ ID NO: 18), as well as primers scs7down280F_G418 (SEQ ID NO: 19) and scs7down280R (SEQ ID NO: 20) were prepared.
SEQ ID NO: 17: 5'-CGAATTCAGCCGAAAACAGTCTTGCTT-3'
SEQ ID NO: 18: 5'-CTCCATGTCGCTTACCACCGCTTTTAGTGC-3'
SEQ ID NO: 19: 5'-CGCTATACTGCAGCCTCGTCCAAAATTGTCA-3'
SEQ ID NO: 20: 5'-CGAATTCTTGCCAACCTGATCTGTGAA-3'
The primer pairs were used to perform PCR with a routinely prepared yeast genomic DNA as a template to give PCR products corresponding to an upstream region of about 280 bp and a downstream region of about 280 bp of the SCS7 gene, respectively.

Next, in the second PCR, these two PCR fragments were mixed and applied to a G-50 gel filtration column (Quick Spin Columns for radiolabeled DNA purification, Roche) to remove the existing primers, and then pFA6a-kanMX4 (EMBLAJ002680) vector having the geneticin (G418) resistance gene as a marker was used as a template to amplify a PCR fragment of about 2.3 kb using a combination of the above primers scs7up280F and scs7down280R. This final PCR product was routinely transformed into the yeast SUR2 disruption strain of Example 2. Transformants were screened on YPD plates (1% yeast extract, 2% polypeptone, 2% glucose, 2% agar) containing 300 mg/L G418 to give a SUR2/SCS7 double disruption strain.

To examine whether the G418 gene was inserted at a desired site and the SCS7 gene was disrupted, primer SCS7 GD CheckF2 (SEQ ID NO: 21) was prepared in the SCS gene, while primer G418CheckR (SEQ ID NO: 22) was prepared in the G418 gene. A transformant giving a 1.2-kb fragment by PCR amplification was identified as a gene disruption strain.

SEQ ID NO: 21: 5'-GCGCTGCATACATAGACATATACAC-3'
SEQ ID NO: 22: 5'-ATACGCGATCGCTGTTAAAAGGACA-3'

### Example 4: Preparation of triple disruption strain of yeast SUR2, yeast alkaline dihydroceramidase (YDC1), and SCS7 genes

Based on a sequence (SEQ ID NO: 43) containing the nucleotide sequence of the yeast alkaline dihydroceramidase gene (YDC1) (SEQ ID NO: 9) and its upstream and downstream regions in a public yeast genome database (SGD), primers ydc1up280F (SEQ ID NO: 23) and ydc1up280R (SEQ ID NO: 24), as well as primers ydc1down250F (SEQ ID NO: 25) and ydc1down250R (SEQ ID NO: 26) were prepared.

SEQ ID NO: 23: 5'-CGAATTCCCCAGAGGCAAAGATGTTA-3'
SEQ ID NO: 24: 5'-TGGATGGCACGGATCCGAAAGGCACACCTGTCATTATGG-3'
SEQ ID NO: 25: 5'-TGTGCCTTTCGGATCCGTGCCATCCATTTGAATC-3'
SEQ ID NO: 26: 5'-CGAATTCCTTTTATGATGGGAGTAACTGCT-3'
The primer pairs were used to perform PCR with a routinely prepared yeast genomic DNA as a template, thereby giving PCR products corresponding to an upstream region of about 280 bp and a downstream region of about 250 bp of the YDC1 gene, respectively. The primers ydc1up280R and ydc1down250F have complementary sequences and contain a BamHI site at the boundary between the upstream and downstream regions of the YDC1 gene.

The PCR products corresponding to the upstream region of about 280 bp and downstream region of about 250 bp of the YDC1 gene were mixed to perform PCR using the primers ydc1up280F and ydc1down250R, thereby giving a 530-bp PCR product containing the upstream and downstream regions of the YDC1 gene flanking the BamHI site. This product was cloned into a vector obtained by self-ligation of pUC19 that had been digested with BamHI and then blunt-ended with T4 DNA polymerase, by use of EcoRI sites contained in the primers ydc1up280F and ydc1down250R (pYDC1-1).

1) Since the hygromycin resistance gene to be introduced subsequently contains an EcoRI site, to remove the EcoRI site in pYDC1-1, primers YDC1up280F_Sse (SEQ ID NO: 27) and YDC1down250R_Sse (SEQ ID NO: 28) were prepared and used to perform PCR with pYDC1-1 as a template. The resulting 530-bp fragment was digested with a restriction enzyme Sse8387I and then purified by agarose gel electrophoresis (GENECLEAN Turbo, Q-BIO Gene). The collected fragment was inserted into the PstI site of pUC19 (pYDC1-2). To ensure excision of the hygromycin resistance gene with a restriction enzyme BglII, primers GA32-2 (SEQ ID NO: 29) and GA32R (SEQ ID NO: 30) were prepared and used to perform PCR with pGA32 (Goldstein et al., yeast. 15 1541 (1999)) as a template to give a 1.5-kb fragment.

SEQ ID NO: 27:
   5'-AAACCTGCAGGCCCAGAGGCAAAGATGTTAAGTTAGATTTTC-3'
SEQ ID NO: 28:
   5'-TTTCCTGCAGGCTTTTATGATGGGAGTAACTGCTGCATGTGT-3'
SEQ ID NO: 29: 5'-TAAGATCTGACATGGAGGCCCAGAATAC-3'
SEQ ID NO: 30: 5'-TAAGATCTCGCACTTAACTTCGCATCTG-3'
This fragment was introduced into the BamHI site of the above pYDC1-2 to eventually complete a construct containing the upstream and downstream regions of the YDC1 gene separated by the hygromycin resistance gene (pYDC1-3). pYDC1-3 was digested with a restriction enzyme Sse8387I to remove the pUC19 vector segment, and the remainder region was purified by agarose gel electrophoresis as described above. The resulting fragment was routinely transformed into the yeast SUR2/SCS7 disruption strain of Example 3. Transformants were screened on YPD plates containing 200 mg/L hygromycin to obtain a SUR2/SCS7/YDC1 triple disruption strain.

2) Alternatively, plasmid pYDp-H (Berben et al., Yeast, 7, 475, 1991) was digested with BamHI, and a fragment of about 1.2 kb containing the HIS3 gene was inserted into the BamHI site of the plasmid containing the upstream and downstream regions of the YDC1 gene. A fragment obtained by digesting the resulting plasmid containing the HIS3 gene flanked by the upstream and downstream regions of the YDC1 gene with EcoR1 was routinely transformed into the yeast SUR2 disruption strain of Example 2. Screening on complete minimal plate lacking histidine (SC-His) gave a SUR2/YDC1 double disruption strain. Next, the PCR product of about 2.3 kb obtained in Example 3, which carries the geneticin (G418) resistance gene, was routinely transformed into the above yeast SUR2/YDC1 double disruption strain. Transformants were screened on YPD plates containing 300 mg/L G418 to obtain a SUR2/SCS7/YDC1 triple disruption strain.

Primers YDC1UPcheckF2 (SEQ ID NO: 31) and ydc1_GD_CheckR2 (SEQ ID NO: 32) were prepared to be amplified by PCR into fragments of different lengths depending on whether the gene is normal or disrupted, and gene disruption was confirmed by PCR.

SEQ ID NO: 31: 5'-CTCGTCCTCTGAACCAAAGC-3'
SEQ ID NO: 32: 5'-GGTAAATAGAACTTTTATGTGCCGC-3'

### Example 5: Preparation of expression vector for endoplasmic reticulum localization signal-attached human DES1 (hDES1m)

A vector was constructed in the following manner, such that the C-terminal amino acid sequence VKKEK (endoplasmic reticulum localization signal) of SUR2 protein, which is a yeast endoplasmic reticulum localization enzyme, was attached to the C-terminal side of hDES1.

The hDES1 expression vector of Example 1 was used as a template for the first PCR to amplify a PCR fragment of about 1.5 kb using a combination of primers 426SseF (SEQ ID NO: 33) and VKKEK_R2 (SEQ ID NO: 36), as well as a fragment of about 0.6 kb using a combination of primers VKKEK_F2 (SEQ ID NO: 35) and 426SseR (SEQ ID NO: 34).

SEQ ID NO: 33: 5'-AACCTGCAGGTGGAATTGTGAGCGGATA-3'
SEQ ID NO: 34: 5'-TTCCTGCAGGTTTTCCCAGTCACGACGTT-3'
SEQ ID NO: 35: 5'-ATGGTGCTGGAGGTAAAGAAAGAGAAATAAATATCATTAGTGCCAAAG-3'
SEQ ID NO: 36: 5'-TAATGATATTTATTTCTCTTTCTTTACCTCCAGCACCATCTCTCCTTT-3'
Next, in the second PCR, these two PCR fragments were mixed and purified by agarose gel electrophoresis (GENECLEAN Turbo, Q-BIO Gene) to remove the existing primers, and the fragment mixture thus purified was used as a template to amplify a PCR fragment of about 2 kb using a combination of the primers 426SseF and 426SseR. This 2-kb fragment was subcloned into pCR-Blunt II-TOPO vector (Invitrogen).

Nucleotide sequencing by the Sanger method was performed to confirm the nucleotide sequence of the clone (SEQ ID NO: 37). In this sequence, the nucleotide sequence of the DES1m ORF region is given as SEQ ID NO: 3, while an amino acid sequence encoded by SEQ ID NO: 3 is given as SEQ ID NO: 4. A fragment of about 1.3 kb was obtained by digestion with restriction enzymes BamHI and XhoI, and inserted into the same restriction enzyme sites of pRS426(2 µ)GPD vector carrying Ura3 as a marker (Mumberg et al., GENE, 156, 119-122, 1995) (phDES1m).

### Example 6: Preparation of transformant strains of ceramide synthetic/metabolic system carrying expression plasmid for human DES1 or endoplasmic reticulum localization signal-attached human DES1 (DES1m)

A strain expressing the DES1 gene (Example 1) or the DES1m gene (Example 5) was constructed using each gene disruption strain obtained in Examples 3 and 4 above.

I. SUR2/SCS7 double disruption (Example 3) + DES1 gene expression
II. SUR2/SCS7 double disruption (Example 3) + DES1m gene expression
III. SUR2/SCS7 double disruption (Example 3) + empty vector
IV SUR2/SCS7/YDC1 triple disruption (Example 4) + DES1 gene expression
V. SUR2/SCS7/YDC1 triple disruption (Example 4) + DES1m gene expression
VI. SUR2/SCS7/YDC1 triple disruption (Example 4) + empty vector
The expression plasmid for human DES1 prepared in Example 1 or endoplasmic reticulum localization signal-attached human DES1 (DES1m) prepared in Example 5 was routinely transformed into the disruption strains obtained in Examples 3 and 4. Transformants were selected on complete minimal plates lacking uracil (SC-Ura).

The expression of the human DES1 gene in the resulting transformant strains was verified by reverse transcription reaction of total RNA purified by an RNeasy kit (Qiagen) using Superscript II (Invitrogen) with primer Hdes1_R (SEQ ID NO: 38) and PCR (RT-PCR) of the reaction product using Ex-taq (Takara Bio) with Hdes1_F (SEQ ID NO: 39) and Hdes1_R (SEQ ID NO: 38).

SEQ ID NO: 38: 5'-TCCAGCACCATCTCTCCTTT-3'
SEQ ID NO: 39: 5'-AGTGGGTCTACACCGACCAG-3'

### Example 7: Ceramide analysis using tritiated (³H) D-erythro-dihydrosphingosine

The above transformant strains of ceramide synthetic/metabolic pathway obtained in Example 6 were cultivated in uracil-free liquid minimal medium containing histidine, leucine, lysine, adenine and tryptophan at 25°C with shaking at 150 rpm for 24 hours, and then the yeast cells were harvested and suspended in liquid minimal medium to prepare 0.5 ml suspensions (16 OD₆₀₀ units/ml). Each suspension was incubated with 10 µl (10 µCi) of tritiated (³H) D-erythro-dihydrosphingosine overnight at 25°C (Zanolari et al., The EMBO Journal, 19, 2824, 2000). The reaction was quenched with 200 µl of 250 mM NaF and 250 mM NaN₃, and then washed three times with ice-cooled sterilized water, and the cells were suspended in 66 µl of sterilized water.

The suspension was vigorously stirred with glass beads to disrupt the cells. Lipids were extracted by adding chloroform and methanol in a ratio of chloroform : methanol : suspension of 10:10:3. The extracts were centrifuged and the resulting supernatants were collected and concentrated/dried by blowing with nitrogen gas. The samples were dissolved in 100 µl of chloroform-methanol-water (10:10:3), and reacted with 20 µl of a 0.6 N solution of NaOH in methanol at 30°C for 90 min, and then neutralized with a 0.6 N acetic acid solution. The reaction solution was desalted by butanol extraction, and the resulting butanol layer (upper layer) was concentrated/dried by blowing with nitrogen gas.

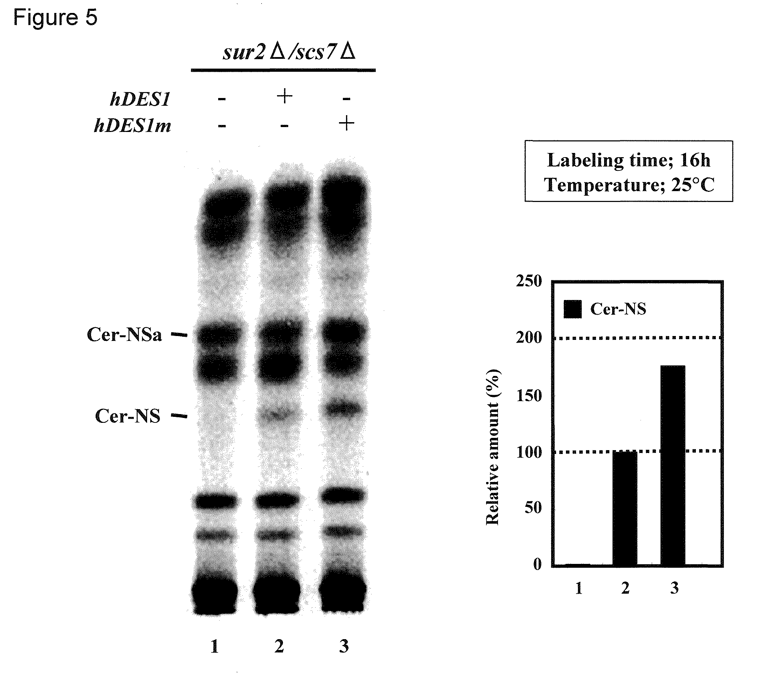

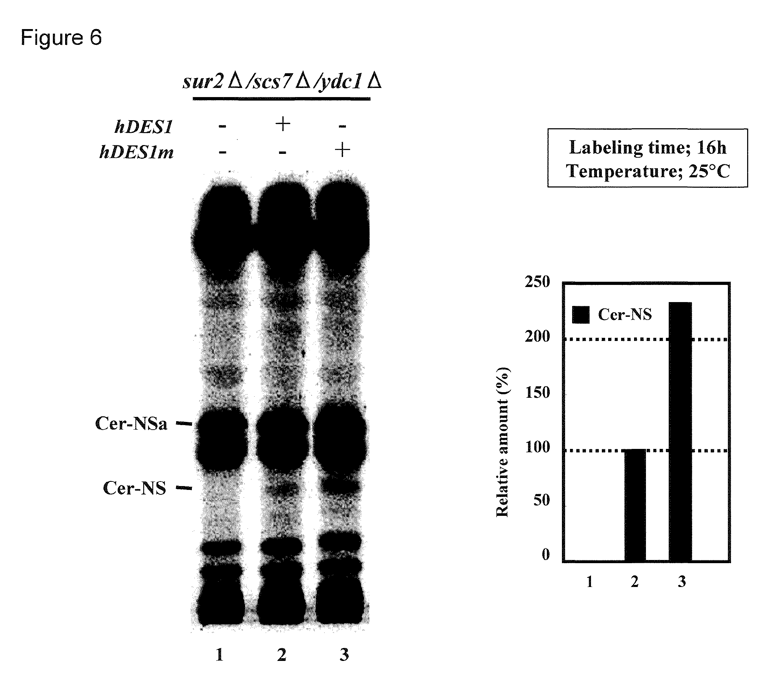

The lipids were dissolved in 20 µl of chloroform-methanol (1:1), spotted on thin layer chromatography (TLC) plates, which had been treated with borate (70 mM Na₂B₄O₇·10H₂O in methanol) for 30 minutes and then dried, and developed with chloroform-methanol (9:1) (Triola et al., Molecular Pharmacology, 66, 1671, 2004). After the development, radioactively labeled ceramides were analyzed by a Bioimage Analyzer (BAS). The results are shown in Figures 5 and 6.

If ceramide NS (CerNS) in the SUR2/SCS7 double disruption or SUR2/SCS7/YDC1 triple disruption strain carrying the expression plasmid for human DES1 is assumed to be 100%, ceramide NS (CerNS) was increased as follows: 177% in the yeast SUR2/SCS7 double disruption strain expressing endoplasmic reticulum localization signal-attached human DES1 (DES1m), and 232% in the yeast SUR2/SCS7/YDC1 triple disruption strain expressing endoplasmic reticulum localization signal-attached human DES1 (DES1m).

### Example 8: Preparation of expression vector for EGFP-attached human DES1 (EGFP-hDES1) or EGFP- and endoplasmic reticulum localization signal-attached human DES1 (EGFP-hDES1m)

To examine at which site(s) in yeast cells hDES1m (composed of hDES1 and an endoplasmic reticulum localization signal attached thereto) is localized, a construct having EGFP attached to the N-terminal side of hDES1 was prepared for each case in this example.

Procedures for vector construction are shown below. From phDES1 of Example 1, the DES1 gene was excised with restriction enzymes BamHI and XhoI, and then inserted into the same restriction enzyme sites of pRS314-GAL-EGFP-EBP2 vector (which is a vector prepared from pRS314 expression vector carrying GA11 promoter and TDH terminator by inserting the EGFP gene into the EcoRI and BamHI sites and the EBP2 gene into the BamHI and XhoI sites; provided by Dr. Keiko Mizuta, Hiroshima University) (pRS314-GAL-EGFP-DES1). The EGFP-DES1 gene was excised with restriction enzymes EcoRI and XhoI, and subcloned into the gene expression vector for yeast pRS426-GPD (Mumberg et al., Gene, 156, 119, 1995) (pEGFP-hDES1).

From pEGFP-hDES1, an EGFP gene region of about 750 bp was excised with restriction enzymes EcoRI and BamHI, and the resulting fragment (EGFP-BamHI) was inserted into the BamHI site of the hDES1m expression vector (phDES1m) obtained in Example 5 (pEGFP-hDES1m).

### Example 9: Preparation of transformant strains carrying expression vector for EGFP-attached human DES1 (EGFP-hDES1) or EGFP- and endoplasmic reticulum localization signal-attached human DES1 (EGFP-hDES1m) for use in DES1 protein localization analysis

The SUR2 disruption strain and SUR2/SCS7 double disruption strain obtained in Examples 2 and 3 and the strain FK113 shown in Example 2 were each transformed with the expression vector for EGFP-hDES1 gene (Example 8) or EGFP-hDES1m gene (Example 8) in a routine manner. Transformants were screened on uracil-free minimal plates containing histidine, leucine, lysine, adenine and tryptophan.

I. Strain FK113 (Example 2) + EGFP-hDES1 gene expression
II. SUR2 disruption strain (Example 2) + EGFP-hDES1 gene expression
III. SUR2/SCS7 double disruption strain (Example 3) + EGFP-hDES1 gene expression
IV. Strain FK113 (Example 2) + EGFP-hDES1m gene expression
V. SUR2 disruption strain (Example 2) + EGFP-hDES1m gene expression
VI. SUR2/SCS7 double disruption strain (Example 3) + EGFP-hDES1m gene expression

### Example 10: DES1 protein localization analysis on transformant strains carrying expression vector for EGFP-attached human DES1 (EGFP-hDES1) or EGFP- and endoplasmic reticulum localization signal-attached human DES1 (EGFP-hDES1m)

The strains obtained in Example 9, i.e., I. EGFP-hDES1 gene-expressing yeast strain FK113, II. EGFP-hDES1 gene-expressing yeast SUR2 disruption strain, III. EGFP-hDES1 gene-expressing yeast SUR2/SCS7 double disruption strain, IV. EGFP-hDES1m gene-expressing yeast strain FK113, V EGFP-hDES1m gene-expressing yeast SUR2 disruption strain, and VI. EGFP-hDES1m gene-expressing yeast SUR2/SCS7 double disruption strain were each cultivated overnight with shaking at 25°C in uracil-free liquid minimal medium containing histidine, leucine, lysine, adenine and tryptophan. The localization of the EGFP fusion proteins was observed under a fluorescent microscope (OLMPUS BX51) with a GFP filter.

The results are shown in Figure 7. hDES1m was confirmed to be localized within the endoplasmic reticulum in each strain.

## Claims

1. A method for producing human ceramide in a yeast cell, which comprises:
1) introducing, by transformation of the yeast cell, an endoplasmic reticulum localization signal-attached sphingolipid Δ4-desaturase gene (DES1m) in which a nucleotide sequence encoding a yeast endoplasmic reticulum localization signal is added to the 3'-end of the sphingolipid Δ4-desaturase gene (DES1); and
2) abolishing, by transformation of the yeast cell, the expression of the yeast sphinganine C4-hydroxylase gene (SUR2).

2. The method according to claim 1, wherein the endoplasmic reticulum localization signal has an amino acid sequence composed of 4 or 5 amino acid residues including two or more lysine residues.

3. The method according to claim 1 or 2, wherein the endoplasmic reticulum localization signal has an amino acid sequence represented by VKKEK (wherein V denotes a valine residue, K denotes a lysine residue, and E denotes a glutamic acid).

4. The method according to any one of claims 1 to 3, wherein the sphingolipid Δ4-desaturase gene (DES1) encodes a protein having the amino acid sequence of SEQ ID NO: 2 or an amino acid sequence obtained by deletion, addition or substitution of one or more amino acid residues in SEQ ID NO: 2, and having sphingolipid Δ4-desaturase activity.

5. The method according to any one of claims 1 to 4, wherein the endoplasmic reticulum localization signal-attached sphingolipid Δ4-desaturase gene (DES1m) encodes a protein consisting of the amino acid sequence of SEQ ID NO: 4.

6. The method according to any one of claims 1 to 5, wherein the yeast sphinganine C4-hydroxylase gene (SUR2) encodes a protein having the amino acid sequence of SEQ ID NO: 6 or an amino acid sequence obtained by deletion, addition or substitution of one or more amino acid residues in SEQ ID NO: 6, and having sphinganine C4-hydroxylase activity.

7. The method according to any one of claims 1 to 6, which further comprises abolishing, by transformation of the yeast cell, the expression of the yeast sphingolipid α-hydroxylase gene (SCS7).

8. The method according to claim 7, wherein the yeast sphingolipid α-hydroxylase gene (SCS7) encodes a protein having the amino acid sequence of SEQ ID NO: 8 or an amino acid sequence obtained by deletion, addition or substitution of one or more amino acid residues in SEQ ID NO: 8, and having sphingolipid α-hydroxylase activity.

9. The method according to any one of claims 1 to 8, which further comprises abolishing, by transformation of the yeast cell, the expression of the yeast alkaline dihydroceramidase gene (YDC1).

10. The method according to claim 9, wherein the yeast alkaline dihydroceramidase gene (YDC1) encodes a protein having the amino acid sequence of SEQ ID NO: 10 or an amino acid sequence obtained by deletion, addition or substitution of one or more amino acid residues in SEQ ID NO: 10, and having alkaline dihydroceramidase activity.

11. The method according to any one of claims 1 to 10, wherein the yeast is selected from yeast species of the genus Saccharomyces.
